(19) Europäisches Patentamt
European Patent Office
Office européen des brevets

(11) EP 0 736 012 B1

# (12) EUROPÄISCHE PATENTSCHRIFT

(45) Veröffentlichungstag und Bekanntmachung des Hinweises auf die Patenterteilung:
**22.11.2000 Patentblatt 2000/47**

(21) Anmeldenummer: **95904470.2**

(22) Anmeldetag: **14.12.1994**

(51) Int. Cl.7: **C07D 217/14**, A61K 31/47, C07D 409/06, C07D 409/12, C07D 409/14, C07D 495/04, C07D 405/12 // (C07D495/04, 333:00, 221:00)

(86) Internationale Anmeldenummer:
**PCT/EP94/04148**

(87) Internationale Veröffentlichungsnummer:
**WO 95/17388 (29.06.1995 Gazette 1995/27)**

## (54) ANELLIERTE DIHYDROPYRIDINE UND DEREN VERWENDUNG FÜR DIE HERSTELLUNG VON PHARMAZEUTISCHEN ZUBEREITUNGEN

ANELLATED DIHYDROPYRIDINES AND THEIR USE IN THE PRODUCTION OF PHARMACEUTICAL PREPARATIONS

DIHYDROPYRIDINES ANNELEES ET LEUR UTILISATION POUR LA PRODUCTION DE PREPARATIONS PHARMACEUTIQUES

(84) Benannte Vertragsstaaten:
**AT BE CH DE DK ES FR GB GR IE IT LI LU MC NL PT SE**
Benannte Erstreckungsstaaten:
**LT SI**

(30) Priorität: **21.12.1993 DE 4343641**
**21.12.1993 DE 4343684**

(43) Veröffentlichungstag der Anmeldung:
**09.10.1996 Patentblatt 1996/41**

(73) Patentinhaber:
- **Boehringer Ingelheim Pharma KG**
**55218 Ingelheim am Rhein (DE)**
Benannte Vertragsstaaten:
**BE CH DE DK ES FR GR IT LI LU MC NL PT SE AT**
- **Boehringer Ingelheim International GmbH**
**55218 Ingelheim am Rhein (DE)**
Benannte Vertragsstaaten:
**GB IE**

(72) Erfinder:
- **LOESEL, Walter**
**D-55435 Gau-Algesheim (DE)**
- **ROOS, Otto**
**D-55270 Schwabenheim (DE)**
- **ARNDTS, Dietrich**
**D-55437 Appenheim (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 288 048**
**EP-A- 0 358 957**
**WO-A-92/11010**
**WO-A-94/00435**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 736 012 B1

## Beschreibung

Die Erfindung betrifft neue anellierte Dihydropyridinessigsäurederivate, Verfahren zu ihrer Herstellung und diese Verbindungen enthaltende Arzneimittel.

Aus der EP-A 37 934 sind Dihydroisochinoline bekannt geworden. Die dort genannten Verbindungen sind cardioton wirksam und weisen eine kontraktilitätssteigernde und blutdruckbeeinflussende Wirkkomponente auf. Sie sind zur Verbesserung der Gewebsdurchblutung und der Gewebssauerstoffversorgung vorgeschlagen worden. Diese Verwendungsmöglichkeiten beruhen auf der vaskulären Wirksamkeit der Verbindungen. In EP-A 251 194 und EP-A 288 048 ist beschrieben worden, daß carbocyclisch und heterocyclisch anellierte Dihydropyridine cardioprotektive beziehungsweise hirnprotektive Wirkung besitzen und einen völlig neuen Typ Ca-antagonistischer Verbindungen verkörpern. In WO 92/11010 ist die Verwendung solcher Verbindungen für hirnprotektive Mittel, für die Behandlung chronisch inflammatorischer Prozesse und zur Hemmung der Blutgerinnung bzw. Blutplättchenaggregation beschrieben.

Gegenstand der vorliegenden Erfindung sind neue carbocyclisch und heterocyclisch anellierte Dihydropyridine und die pharmazeutische Verwendung dieser Verbindungen. Die neuen Verbindungen haben wertvolle therapeutisch nutzbare Eigenschaften. Sie sind als cardioprotektive Mittel, als hirnprotektive Mittel (insbesondere bei der Behandlung von Patienten, die einen Schlaganfall erlitten haben oder gefährdet sind, einen Schlaganfall zu erleiden), als Mittel für die Behandlung chronisch inflammatorischer Prozesse (z.B. Bronchialasthma, Arthritis) verwendbar. Ferner können diese Verbindungen als Mittel mit antiproliferativer Wirkung und als Mittel zur Behandlung der Colitis Ulcerosa und des Morbus Crohn verwendet werden.

Die Erfindung betrifft Verbindungen der allgemeinen Formel I

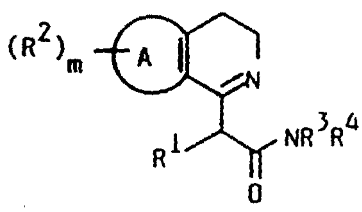

I

worin

A einen Benzo, Indolo oder Thienorest bedeutet;
worin, wenn A Benzo ist, m 2 oder 3 ist, (vorzugsweise 2, wobei die beiden $R^2$ in den Positionen 6 und 7 sind) und die Substituenten $R^2$ unabhängig voneinander Hydroxy, $(C_1-C_4)$Alkoxy, Benzyloxy, Halogen (F, Cl, Br, J), $(C_1-C_4)$Alkyl, Methansulfonyloxy oder Methansulfonamido, oder zwei benachbarte Substituenten $R^2$ zusammen $-O-CH_2-O-$ oder $-O-CH_2-CH_2-O-$ sein können; und wenn A Indolo oder Thieno ist, m Null ist;

$R^1$ Thienyl oder die Gruppe

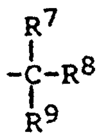

bedeutet,
worin

$R^7$, $R^8$ und $R^9$ unabhängig voneinander Methyl, Ethyl, Propyl, Phenyl oder Benzyl sein können, wobei nicht mehr als 2 der Substituenten gleichzeitig Phenyl oder Benzyl sein können;

$R^3$ und $R^4$ unabhängig voneinander eine der folgenden Bedeutungen haben

(a) Wasserstoff,

(b) verzweigtes oder unverzweigtes Alkenyl mit 3 bis 6 Kohlenstoffatomen,

(c) verzweigtes oder unverzweigtes Alkinyl mit 3 bis 6 Kohlenstoffatomen, oder

(d) verzweigtes oder unverzweigtes Alkyl mit 1-12 Kohlenstoffatomen bedeuten, wobei das Alkyl substituiert sein kann durch

Hydroxy,
$(C_{1-4})$Alkoxy,
Di$(C_1$-$C_4)$Alkylamino,
Furyl,
Pyridyl,
Pyrrolidinyl, N-Methylpyrrolidinyl,
Morpholino,
Indolyl,
Nitrilo,
Thienyl,
Adamantyl,
Cyclohexyl,
Phenoxy,
Naphthyloxy oder Phenyl, [wobei dieses Phenyl oder das in der Phenoxygruppe enthaltene Phenyl ein-, zwei- oder dreifach durch Hydroxy, $(C_1$-$C_4)$Alkoxy, Benzyloxy, Halogen (F, Cl, Br, J), $CF_3$, $N_3$, CN, $(C_1$-$C_4)$Alkyl, Adamantyl, $-SO_2NH_2$, $-NHCOCH_3$, $-NHSO_2CH_3$ oder $CH_3SO_2O$- oder durch die Brücke $-O-CH_2-O-$ substituiert sein kann;] oder durch zwei unsubstituierte Phenylgruppen;
oder $R^3$ Wasserstoff bedeutet und $R^4$ Cyclohexyl, Phenyl [wobei dieses Phenyl ein-, zwei- oder dreifach durch Hydroxy, $(C_1$-$C_4)$Alkoxy, Benzyloxy, Halogen (F, Cl, Br, J), $CF_3$, $N_3$, $(C_1$-$C_4)$Alkyl, Adamantyl, $-SO_2NH_2$, $-NHCOCH_3$, $-NHSO_2CH_3$ oder $CH_3SO_2O$- oder durch die Brücke $-O-CH_2-O-$substituiert sein kann] Pyridyl oder N-Benzylpiperidyl;
oder $R^3$ und $R^4$ gemeinsam mit dem Stickstoffatom, an das sie gebunden sind,
Pyrrolidinyl,
Piperidinyl,
Morpholinyl, Thiomorpholinyl
oder
Piperazinyl bedeuten, wobei der Piperazinylring gegebenenfalls durch Methyl, unsubstituiertes Phenyl, Mono- oder Di$(C_1$-$C_4)$alkoxyphenyl, Cyanosubstituiertes Phenyl, Pyrimidinyl, Phenyl$(C_1$-$C_4)$alkyl, $(C_1$-$C_4)$Alkylphenyl oder

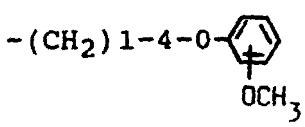

N-substituiert sein kann;

oder deren Salze mit physiologisch verträglichen Säuren oder Komplexbildnern.

Verbindungen der Formel I bilden Tautomere der Formel II

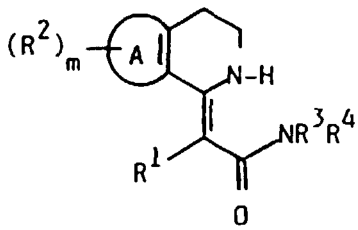

II

Die Tautomeren sind nach bekannten Methoden trennbar z.B. durch Säulenchromatographie oder selektive Reduktion ($NaBH_4$ beziehungsweise katalytische Reduktion).

Die Verbindungen der Formel II können in cis und/oder trans-Form vorliegen:

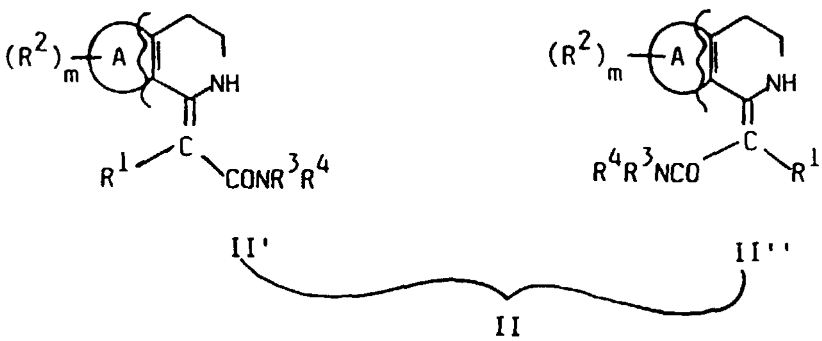

II' II''

II

Wenn die Struktur einer Verbindung nicht ausdrücklich angegeben ist, ist die Angabe der Formel I so zu verstehen, daß Struktur II ebenfalls umfaßt wird.

In den im Text verwendeten Definitionen können die Reste und Gruppen jeweils gleich oder verschieden sein, d.h. wenn einer der zuvor genannten Substituenten in einem bestimmten Molekül mehrfach vorkommt, kann die jeweilige Bedeutung im Rahmen der Definitionsbreite frei gewählt werden.

Mit Alkyl sind insbesondere $C_1$-$C_6$-Alkyl- und $C_1$-$C_4$-Alkylradikale gemeint die ihrerseits substituiert sein können oder als Alkylradikale Bestandteil einer funktionellen Gruppe - wie Alkoxy oder Alkylthio - sind. Die Alkylradikale umfassen die Methyl-, Ethyl-, n-Propyl-, iso-Propyl-, n-Butyl-, sek-Butyl-, iso-Butyl-, tert-Butylradikale sowie die verschiedenen isomeren Pentyl- und Hexylradikale, wie etwa das Isopentyl, das Neopentyl, das n-Pentyl und das n-Hexylradikal.

Die vorstehende Definition gilt somit auch wenn das Alkylradikal seinerseits substituiert und/oder selbst Bestandteil einer Alkoxyalkyl-, Alkoxycarbonyl-, Alkoxy-, Alkylthio-, Alkylsulfonyl-, Monoalkylamino-, Alkylmethyl-, Alkylthiomethyl-, Dialkylaminogruppe ist oder das Alkylradikal als Substituent an ein aromatisches heterocyclisches oder carboxyclisches System gebunden ist.

Halogene sind Fluor, Chlor, Brom und Jod, vorzugsweise Fluor, Chlor und Brom und in zweiter Linie Jod.

$C_3$-$C_6$-Cycloalkyl sind Cyclopropan, Cyclobutan, Cyclopentan und Cyclohexan.

$C_5$-$C_6$-Cycloalkene sind z.B. Cyclopenten, Cyclohexen und Cyclohexadien.

$C_3$-$C_6$-Alkine sind die isomeren Hexine, Pentine, Butine und Propine bevorzugt ist Propargyl

$C_3$-$C_6$-Alkene sind die isomeren Hexene, Pentene, Butene und Propene bevorzugt ist Allyl.

Ein bevorzugter Aspekt der Erfindung sind Verbindungen der allgemeinen Formel I worin

A einen Benzo- oder Thienorest bedeutet;
worin, wenn A Benzo ist, m 2 ist, die beiden $R^2$ in den Positionen 6 und 7 sind und unabhängig voneinander Hydroxy, ($C_1$-$C_4$)Alkoxy, Benzyloxy, Halogen (F, Cl, Br, J), ($C_1$-$C_4$)Alkyl, Methansulfonyloxy oder Methansulfonamido, oder zwei benachbarte Substituenten $R^2$ zusammen -0-$CH_2$-0- oder -0-$CH_2$-$CH_2$-O- sein können; und

wenn A Thieno ist, m Null ist;

$R^1$ Thienyl oder die Gruppe

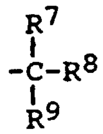

bedeutet,
worin

$R^7$, $R^8$ und $R^9$ unabhängig voneinander Methyl, Ethyl, Propyl, Phenyl oder Benzyl sein können, wobei nicht mehr als 2 der Substituenten gleichzeitig Phenyl oder Benzyl sein können;

$R^3$ und $R^4$ unabhängig voneinander

(a) Wasserstoff,
(b) verzweigtes oder unverzweigtes Alkenyl mit 3 bis 6 Kohlenstoffatomen,
(c) verzweigtes oder unverzweigtes Alkinyl mit 3 bis 6 Kohlenstoffatomen, oder
(d) verzweigtes oder unverzweigtes Alkyl mit 1-12 Kohlenstoffatomen bedeuten, wobei das Alkyl substituiert sein kann durch

Hydroxy,
$(C_1$-$C_4)$Alkoxy,
Di$(C_1$-$C_4)$Alkylamino,
Furyl,
Pyridyl,
Pyrrolidinyl, N-Methylpyrrolidinyl,
Morpholino,
Indolyl,
Nitrilo,
Thienyl,
Adamantyl,
Cyclohexyl,
Phenoxy,
Naphthyloxy oder Phenyl, wobei dieses Phenyl oder das in der Phenoxygruppe enthaltene Phenyl ein-, zwei- oder dreifach durch Hydroxy, $(C_1$-$C_4)$Alkoxy, Benzyloxy, Halogen (F, Cl, Br, J), $CF_3$, $N_3$, $(C_1$-$C_4)$Alkyl, Adamantyl, $-SO_2NH_2$ oder $-NHCOCH_3$ oder durch die Brücke $-O-CH_2-O-$ substituiert sein kann;
oder $R^3$ Wasserstoff bedeutet und $R^4$ Cyclohexyl, Phenyl, Fluorophenyl, Pyridyl oder N-Benzylpiperidyl;
oder $R^3$ und $R^4$ gemeinsam mit dem Stickstoffatom, an das sie gebunden sind,
Pyrrolidinyl,
Piperidinyl,
Morpholinyl, Thiomorpholinyl
oder
Piperazinyl bedeuten, wobei der Piperazinylring gegebenenfalls durch Methyl, unsubstituiertes Phenyl, Mono- oder Di$(C_1$-$C_4)$alkoxyphenyl, Pyrimidinyl, Phenyl$(C_1$-$C_4)$alkyl oder

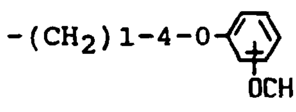

N-substituiert sein kann.

Vorzugsweise steht A für die anellierten Ringsysteme

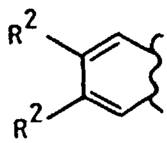 . 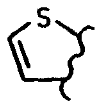 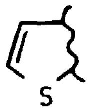

worin $R^2$ wie oben definiert ist.

Ein weiterer bevorzugter Aspekt der Erfindung sind Verbindungen I, worin A Indolo ist und die anderen Substituenten wie oben definiert sind, vorzugsweise $NR^3R^4$ entweder Morpholinyl ist oder in $NR^3R^4$

$R^3$ Wasserstoff ist und $R^4$ Alkyl mit 1 bis 4 Kohlenstoffatomen, das wie oben definiert substituiert sein kann.

Von den Verbindungen I, worin A Benzo ist, sind solche bevorzugt, worin m 2 ist und die beiden $R^2$ unabhängig voneinander Methoxy, Hydroxy, Benzyloxy, Methyl oder Chlor oder zusammen -$OCH_2$0- sind, wobei die beiden $R^2$ in den Positionen 6 und 7 stehen, insbesondere solche Verbindungen, worin $R^2$ Methoxy, Hydroxy, Benzyloxy oder Methyl ist, insbesondere solche worin beide $R^2$ gleich sind und Hydroxy oder Methoxy bedeuten.

Von den Verbindungen I sind solche bevorzugt, worin $R^1$ tert. Butyl ist.

Von den Verbindungen I sind ferner solche bevorzugt, worin $NR^3R^4$ eine der folgenden Bedeutungen hat

a) in $NR^3R^4$ ist $R^3$ Wasserstoff und $R^4$ $C_1$-$C_6$ Alkyl;

b) in $NR^3R^4$ ist $R^3$ Wasserstoff und $R^4$ verzweigtes oder unverzweigtes Alkinyl mit 3 bis 6 (vorzugsweise 3) Kohlenstoffatomen

c) in $NR^3R^4$ ist $R^3$ Wasserstoff und $R^4$ verzweigtes oder unverzweigtes Alkyl mit 1-4 (vorzugsweise 1 bis 3, insbesondere 2) Kohlenstoffatomen, wobei das Alkyl substituiert ist durch

Methoxy,
Dimethylamino,
Pyrrolidinyl, N-Methylpyrrolidinyl,
Morpholino,
Thienyl,
Adamantyl,
Pyridyl,
N-Benzylpiperidyl,
Cyclohexyl,
Phenoxy,
Naphthyloxy oder 1 oder 2 Phenyl, wobei dieses Phenyl (wenn nur eine Phenylgruppe vorliegt) oder das in der Phenoxygruppe enthaltene Phenyl ein-, zwei- oder dreifach durch Methoxy, Ethoxy, Benzyloxy, Halogen (insbesondere Cl, J), $CF_3$, $N_3$, Methyl, tert. Butyl, -$SO_2NH_2$, oder durch die Brücke -O-$CH_2$-O- substituiert sein kann;
oder $R^3$ Wasserstoff bedeutet und $R^4$ Cyclohexyl, Phenyl, Fluorophenyl, Pyridyl oder N-Benzylpiperidyl;

d) in $NR^3R^4$ sind $R^3$ und $R^4$ unabhängig voneinander Methyl, Ethyl, $(CH_2)_{1-4}$ Phenyl (worin die Phenylgruppe substituiert sein kann wie die in (c) angegebene Phenylgruppe)

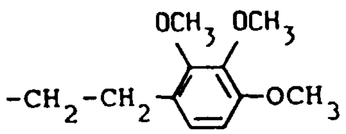 ;

vorzugsweise

e) $R^3$ und $R^4$ gemeinsam mit dem Stickstoffatom, an das sie gebunden sind, sind
Piperidinyl,
Morpholinyl, Thiomorpholinyl,
oder
Piperazinyl, wobei der Piperazinylring gegebenenfalls durch Methyl oder Benzyl N-substituiert sein kann;
insbesondere solche, worin $NR^3R^4$ eine der folgenden Bedeutungen hat

a) in $NR^3R^4$ ist $R^3$ Wasserstoff und $R^4$ $C_2$-$C_6$ Alkyl;
b) in $NR^3R^4$ ist $R^3$ Wasserstoff und $R^4$ ist $CH_2CCH$;
c) in $NR^3R^4$ ist $R^3$ Wasserstoff und $R^4$ verzweigtes oder unverzweigtes Alkyl mit 2-4 Kohlenstoffatomen, wobei das Alkyl substituiert ist durch

Methoxy,
Dimethylamino,
N-Methylpyrrolidinyl,
Thienyl,
Adamantyl,
Phenoxy,
Naphthyloxy oder 1 oder 2 Phenyl, wobei dieses Phenyl (wenn nur eine Phenylgruppe vorliegt) oder das in der Phenoxygruppe enthaltene Phenyl ein-, zwei- oder dreifach durch Methoxy, Ethoxy, $N_3$, Methyl, tert. Butyl oder $-SO_2NH_2$ substituiert sein kann;

d) in $NR^3R^4$ sind $R^3$ und $R^4$ unabhängig voneinander Methyl, Ethyl, $(CH_2)_{1-4}$ Phenyl (worin die Phenylgruppe durch F substituiert sein kann) oder insbesondere

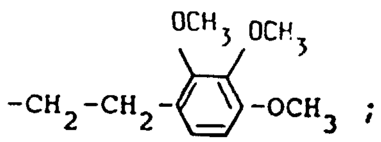

e) $R^3$ und $R^4$ gemeinsam mit dem Stickstoffatom, an das sie gebunden sind, sind
Piperazinyl, N-subsitutiert durch Methyl oder Benzyl.

Besonders hervorzuheben sind Verbindungen I, worin $NR^3R^4$ eine der folgenden Bedeutungen hat.

a) in $NR^3R^4$ ist $R^3$ Wasserstoff und $R^4$ ist Ethyl, tert. Butyl oder
$(CH_2)_{1\ oder\ 2}$-$C(CH_3)_3$;

b) $NR^3R^4$ ist $NHCH_2CCH$;

c) in $NR^3R^4$ ist $R^3$ Wasserstoff und $R^4$ ist Ethyl, Propyl oder Methylpropyl, das durch Phenyl substituiert ist, das ein-, zwei- oder dreifach durch Methyl oder Methoxy oder einfach durch tert. Butyl substituiert sein kann;

d) in $NR^3R^4$ sind $R^3$ und $R^4$ gleich, nämlich

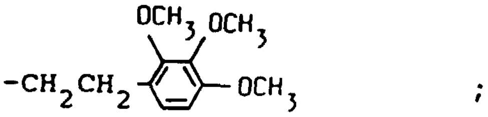

e) $NR^3R^4$ ist

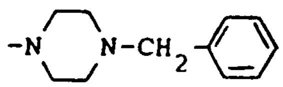 ;

insbesondere solche, worin $R^3$ Wasserstoff oder $(C_1-C_4)$Alkyl-Phenyl ist und $R^4$ $(C_1-C_4)$ Alkyl-Phenyl, wobei in diesen Gruppen jeweils vorzugsweise $C_1$-Alkyl enthalten ist und Phenyl monosubstituiert ist, durch Halogen (vorzugsweise Cl oder F), $CF_3$, Methoxy oder Ethoxy, wobei dieser Substituent vorzugsweise in o-Stellung ist.

Die Verbindungen der Formel I können nach an sich bekannten Verfahren hergestellt werden, vorzugsweise analog zu der in der deutschen Patentanmeldung P 37 18 570.5, EP 358 957, EP 37 934, EP 251 794 und EP 288 048 beschriebenen Methode.

In Gegenwart eines Kondensationsmittels kann ein Malonsäurediamid der allgemeinen Formel IV

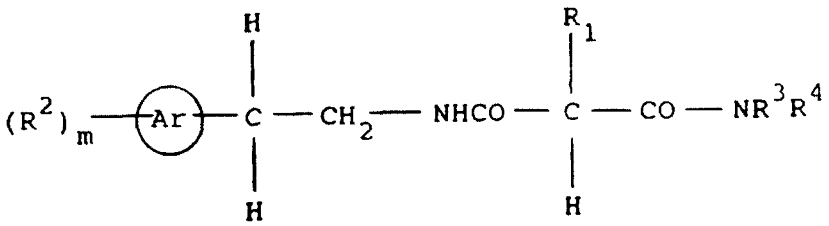

in der $R^1$, $R^2$, $R^3$, $R^4$ und m, wie oben definiert sind und Ar Phenyl, Indolyl oder 2- oder 3-Thienyl bedeutet, zu den entsprechenden Verbindungen cyclisiert werden.

Als Kondensationsmittel für dieses Verfahren eignen sich starke Lewis-Säuren, wie z.B. Phosphoroxychlorid, Phosphorpentachlorid, Phosphortrichlorid, Phosphorpentoxid, Titantetrachlorid, Bortrifluorid, Zinntetrachlorid, aber auch anorganische Säuren, wie z.B. Polyphosphorsäure, Schwefelsäure, Fluorsulfonsäure und Fluorwasserstoffsäure, oder Gemische von Kondensationsmitteln wie z.B. ein Gemisch von Phosphoroxychlorid und Phosphorpentachlorid, oder ein Gemisch von Phosphorpentoxid und $(C_1-C_4)$ Alkylsulfonsäure z.B. mit einem $P_20_5$-Anteil von ca. 10 Gewichtsprozenten.

Die Cyclisierung kann in Gegenwart oder Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen, beispielsweise Benzol, Alkylbenzole (z.B. Toluol, Xylol), Chlorbenzole, Chloroform, Acetonitril, Dekalin. Eine bevorzugte Variante des Verfahrens besteht darin, als Kondensationsmittel Phosphoroxychlorid im Gemisch mit Acetonitril oder ein $(C_1-C_4)$Alkylsulfonsäure-/Phosphorpentoxid-Gemisch, ohne Zusatz von Lösungsmitteln zu verwenden.

Bevorzugt erfolgt die Cyclisierung mit Phosphoroxychlorid/Acetonitril oder in schwierigen Fällen mit einem Gemisch von Phosphorpentoxid und $(C_1-C_4)$Alkylsulfonsäure (bevorzugt Methansulfonsäure).
Die Umsetzung kann innerhalb eines großen Temperaturbereichs, vorzugsweise unter Erwärmen auf 50°C bis etwa den Siedepunkt des Reaktionsgemisches, durchgeführt werden.

Die erforderliche Reaktionsdauer liegt je nach Ausgangsverbindung IV zwischen 2 und 15 Stunden.

Die für diese Herstellung benötigte 3-Thiophenmalonsäure ist im Handel. Die 2-Thiophenmalonsäure kann nach an sich bekannten Verfahren hergestellt werden (z.B. aus 2-Thiophenessigsäure nach der Carbonatmethode oder aus 2-Thiophenbromid und Malonsäurediethylester).

Die Verbindungen der Formel I sind Basen und können auf übliche Weise mit anorganischen oder organischen Säuren sowie Salz- und Komplexbildnern in beliebige physiologisch unbedenkliche Addukte (Salze) überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, Phthalsäure, Zimtsäure, Salicylsäure, Ascorbinsäure, Methansulfonsäure und dergleichen.

Die Verbindungen können oral, parenteral oder topisch verabreicht werden. Der gewünschte therapeutische Dosis ist von der Indikation und Darreichungsform abhängig und kann experimentell bestimmt werden. Geeignete Anwendungsformen sind beispielsweise Tabletten, Kapseln, Zäpfchen, Lösungen, Säfte, Emulsionen, Aerosole oder

dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Die Verbindungen können sowohl enteral als auch parenteral verabreicht werden. Als Dosis für die orale Anwendung werden 0,1 bis 500 mg Wirkstoff pro Dosis, für die i.v.-Anwendung von 0,05 bis 150 mg pro Dosis vorgeschlagen. Der gewünschte therapeutische Dosis ist von der Indikation und Darreichungsform abhängig und kann experimentell bestimmt werden.

Die Arzneimittel sind für orale oder parenterale, gegebenenfalls auch topische Verabreichung geeignet. Als Arzneimittelformen dienen vorwiegend Tabletten, Dragees, Ampullen und Saftzubereitungen. Die Einzeldosis zu diesen Arzneimittelformen beträgt zwischen 1,0 und 200 mg, vorzugsweise 20 und 50 mg pro 75 kg Körpergewicht. Je nach der Schwere des Falles sind täglich im allgemeinen 1 bis 3 Einzeldosen zu verabreichen.

Die folgenden Beispiele sollen die Erfindung näher erläutern:

Beispiel 1

1. tert. Butylmalonsäuremonoethylester

In ein Gemisch aus 21,6 g tert. Butylmalonsäurediethylester, 50 ml Ethanol und 50 ml Wasser wird bei Raumtemperatur während 30 min eine Lösung von 7,6 g KOH (85%ig) in 50 ml Wasser unter Rühren eingetropft. Nach 15 stunden wird das Ethanol im Vakuum abdestilliert. Zum Rückstand werden nach dem Abkühlen 300 ml $CH_2Cl_2$ zugegeben. Es wird mit einer Lösung von 13,6 g $KHSO_4$ in 100 ml $H_2O$ unter Eiskühlung angesäuert und die wäßrige Phase mehrmals mit $CH_2Cl_2$ extrahiert. Die vereinigten organischen Phasen werden mit Wasser gewaschen, über $Na_2SO_4$ getrocknet und bei 30°C Badtemperatur im Vakuum eingeengt. Es verbleiben 15,35 g (= 81,7 % der Theorie) Monoethylester (hellgelbes Öl).

2. tert. Butylmalonsäuremonoethylester-N-[2-(3,4-dimethoxyphenyl)ethyl]-amid

In eine Lösung von 15,35 g tert. Butylmalonsäuremonoethylester in 200 ml wasserfreiem $CH_2Cl_2$ werden bei Raumtemperatur 15,88 g N,N'-Carbonyldiimidazol portionsweise eingerührt. Nach 30 Min. werden 14,8 g 2-(3,4-Dimethoxyphenyl)ethylamin zugegeben. Nach weiteren 15 Stunden wird das Lösungsmittel im Vakuum abdestilliert. Der Rückstand wird mit 200 ml Wasser versetzt, mit 2 N HCl angesäuert und mit Essigester extrahiert. Die organische Phase wird mit Wasser gewaschen und nach dem Trocknen über $Na_2SO_4$ eingeengt. Der Rückstand wird mit Petrolether (40°-60°C) verrieben und zur Kristallisation gebracht. Ausbeute: 24,7 g (86,2 % der Theorie); Fp. 68-70°C.

3. tert. Butylmalonsäure-mono-N-[2-(3,4-dimethoxyphenyl)-ethyl]amid

42 g Esteramid (s.o.) und 180 ml 1 N NaOH werden 2 Stunden am Rückfluß erhitzt. Nach dem Abkühlen und Filtrieren wird die Lösung mit Ether extrahiert und durch Zugabe von 50 ml 4 N HCl angesäuert. Aus der Lösung scheiden sich beim Stehen Kristalle ab. Sie werden abgesaugt und bei 40-50°C getrocknet. Ausbeute 35,4 g (91,3 % der Theorie). Fp. 103-104°C

4. tert. Butylmalonsäure-N-[2-(3,4-dimethoxyphenyl)ethyl]-N'-(3,3-diphenylpropyl)-diamid

In eine Lösung von 3,23 g tert. Butylmalonsäuremonoamid (aus Beispiel 3) in 50 ml waserfreiem $CH_2Cl_2$ werden bei Raumtemperatur 2,1 g N,N'-Carbonyldiimidazol eingetragen. Nach 30 min werden 2,11 g 3,3-Diphenylpropylamin zugegeben. Nach 15 stündigem Stehen bei Raumtemperatur wird das Lösungsmittel abdestilliert. Der Rückstand wird mit Wasser versetzt, mit 2 N HCl angesäuert und mit Ethylacetat extrahiert. Die organische Phase wird nach dem Waschen mit Wasser über $Na_2SO_4$ getrocknet und im Vakuum eingeengt. Der Rückstand (4,9 g ≅ 95 % der Theorie) wird ohne weitere Reinigung in die Cyclisierungsreaktion eingesetzt.

5. (R,S)-(3,4-Dihydro-6,7-dimethoxyisochinolin-1-yl)-2-tert.butyl-N-(3,3-diphenylpropyl)-acetamid

Ein Gemisch aus 5,15 g tert. Butylmalonsäurediamid (aus Beispiel 4), 1,9 ml $POCl_3$ und 35 ml Acetonitril wird 2 Stunden am Rückfluß erhitzt. Nach dem Abkühlen wird auf Eiswasser gegossen, mit gesättigter Sodalösung alkalisch gestellt und mit Essigester extrahiert. Die organische Phase wird mit Wasser gewaschen, über $Na_2SO_4$ getrocknet und eingeengt. Der Rückstand wird in Aceton gelöst, mit der berechneten Menge etherischer Salzsäure in das Hydrochlorid überführt und durch Reiben zur Kristallisation gebracht. Ausbeute: 4,45 g (83,6 % der Theorie); Fp. 147-148°C.

Beispiel 2

3-Thienylmalonsäure-N-[2-(3,4-dimethoxyphenyl)ethyl]-amid

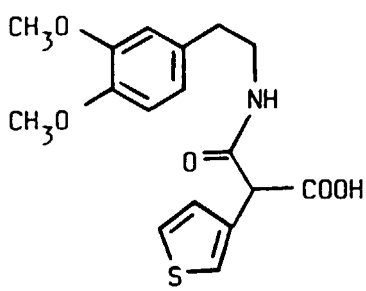

15,1 g (40 Mmol) 3-Thienylmalonsäuremonoethylester-N-[2-(3,4-dimethoxyphenyl)ethyl)-amid werden in 150 ml Methanol und 100 ml Dioxan gelöst und unter Rühren und Eiskühlung 42 ml (42 Mmol) 1N NaOH tropfenweise zugesetzt. Es wird noch 2 Stdn. bei z.T. weitergerührt, die organischen Lösungsmittel werden im Vakuum abdestilliert und der Rückstand wird zwischen Wasser und $CH_2Cl_2$ verteilt.

Die wäßrige Phase wird unter Kühlung und Rühren mit 10%iger Zitronensäure angesäuert und mit $CH_2Cl_2$ extrahiert. Nach Waschen mit Wasser, ges. NaCl-Lösung und Trocknen über $MgSO_4$ wird das Lösungsmittel im Vakuum abdestilliert und dabei ein Rückstand von 12,7 g erhalten. Durch Umkristallisieren aus Methylenchlorid/Methanol/Ether werden 11,7 g (83,7 % der Theorie) der Titelverbindung erhalten.

3-Thienylmalonsäure-N-[2-(3,4-dimethoxyphenyl)ethyl]-N'dibenzyl-di-amid

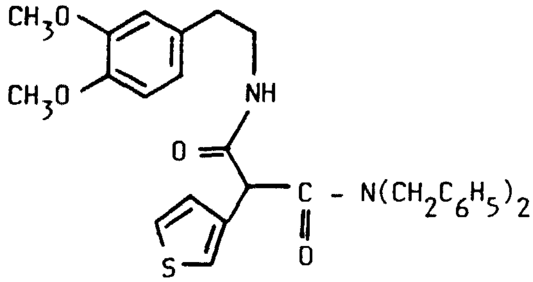

In eine Lösung von 3,75 g (11 Mmol) 3-Thienylmalonsäure-N-[2-(3,4-dimethoxyphenyl)-ethyl)-amid in 100 ml abs. Tetrahydrofuran werden bei 5°C unter Rühren 1,78 (11 Mmol) Carbonyldimidazol portionsweise eingetragen. Das Reaktionsgemisch wird 30 Minuten bei Raumtemperatur gerührt und anschließend 2,17 g (11 Mmol) Dibenzylamin zugegeben. Nach 16-stündigem Rühren bei R.T. wird eingeengt und der Rückstand zwischen Essigester und Wasser verteilt. Die organische Phase wird nacheinander mit Wasser, 5-%iger $KHSO_4$-Lösung, ges. $NaHCO_3$-Lösung, Wasser und gesättigter NaCl-Lösung gewaschen, über $MgSO_4$ getrocknet und das Lösungsmittelgemisch im Vakuum abdestilliert. Der Rückstand wird in wenig Äther umkristallisiert.

Ausbeute: 5,1 g (87,7 % der Theorie) der Titelverbindung werden erhalten.

(R,S)-(3,4-Dihydro-6,7-dimethoxyisochinolin-1-yl)-2-(3-Thienyl)-N,N-dibenzyl-acetamid

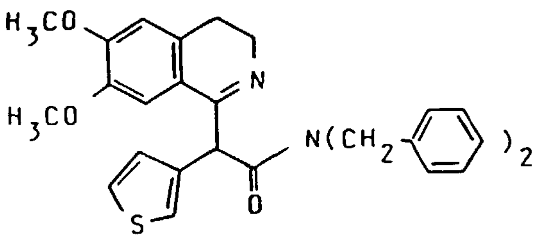

5,1 g (96 Mmol)3-Thienylmalonsäure-N-[2-(3,4-dimethoxyphenyl)ethyl]-N'-dibenzyl-di-amid werden in 50 ml Acetonitril p.A. mit 4,41 g (28,8 Mmol) Phosphoroxychlorid versetzt und 1 Std. unter $N_2$-Atmosphäre am Rückfluß zum Sieden erhitzt. Nach dem Abkühlen werden 150 ml Essigester zugesetzt, mit gesättigter $NaHCO_3$-Lösung neutralisiert, mit Wasser und ges. NaCl-Lösung gewaschen, über $MgSO_4$ getrocknet und die Lösungsmittel im Vakuum abdestilliert. Der Rückstand wird in 10 ml abs. Aceton gelöst, 900 mg (10 Mmol) Oxalsäure zugesetzt und das Salz kristallin nach Zugabe von ca. 50 ml abs. Diethylether ausgefällt.

Ausbeute: 4,8 g (83,3 % der Theorie) der Titelverbindung als Oxalat; Fp.: 128 - 130 °C.

Die folgenden Tabellen fassen Beispiel von erfindungsgemäßen Verbindungen zusammen. Die Herstellung dieser Verbindungen kann analog zu den oben beschriebenen Verfahren erfolgen.

Tabelle 1

| 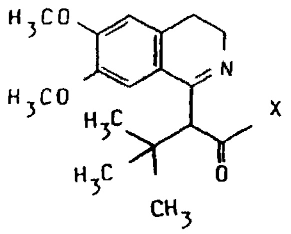 | Fp.(°C) | Salzform | tautomere Struktur |
|---|---|---|---|
| X = 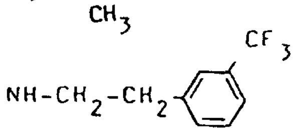 | 76 | Fu(1.5) | |
| 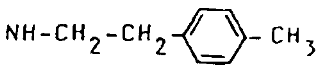 | 105 | Fu | |
| 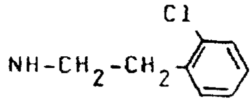 | 152 | Fu(1.5) | |
| 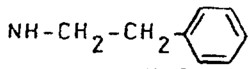 | 230 | Cl | |
| 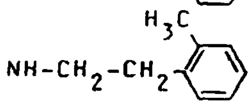 | 138 | Fu(1.5) | |
| 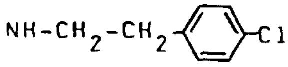 | 112 | Fu | |
| 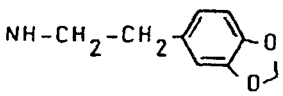 | | | |
| 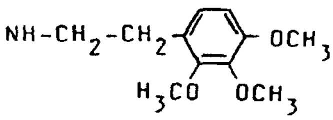 | | | |
| 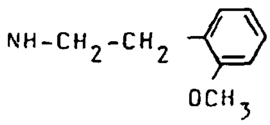 | 98 | Fu(1.5) | |
| 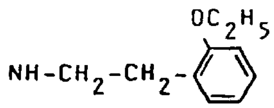 | | | |
| 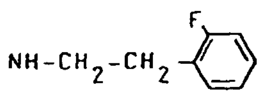 | 209 | Cl | |
| 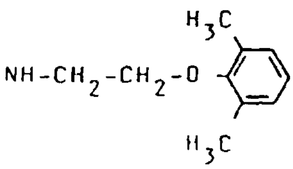 | | | |

| | Fp.(°C) | Salzform | tautomere Struktur |
|---|---|---|---|
| 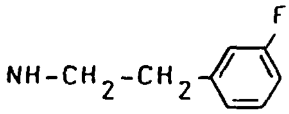 | 215 | Cl | |
| 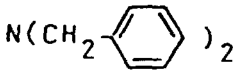 | | | |
| 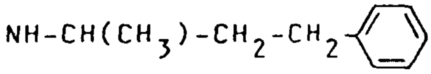 | | | |
| 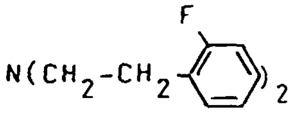 | | | |
| 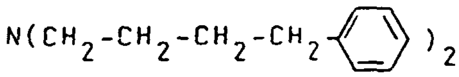 | | | |
| 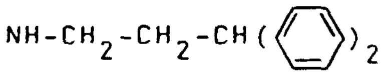 | 174 | Fu | |
| 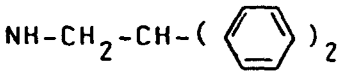 | 208 | Fu | |
| 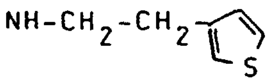 | 232 | Cl | |
| 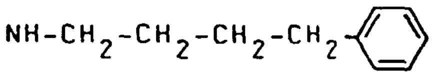 | 165 | Cl | |
| 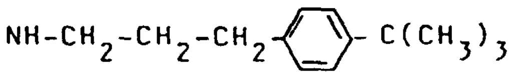 | 133 | Fu(1.5) | I |
| $NH-(CH_2)_9-CH_3$ | 147 | Cl | |
| 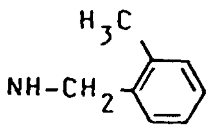 | 210 | Cl | |

| | Fp.(°C) | Salzform | tautomere Struktur |
|---|---|---|---|
| 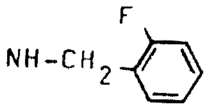 | 223 | Cl | |
| 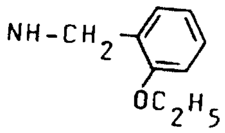 | | | |
| 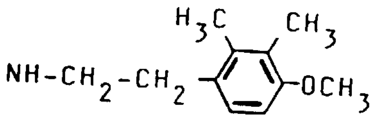 | | | |
| 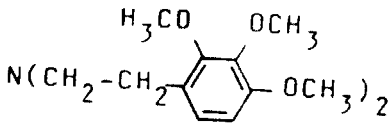 | | | |
| 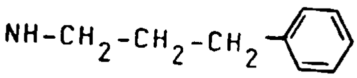 | 209 | Cl | I |
| 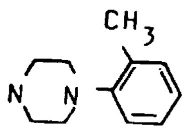 | 187 | Fu(1.5) | |
| 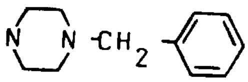 | | | |
| 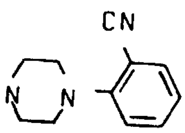 | | | |

| | Fp.(°C) | tautomere Struktur | Salz-form |
|---|---|---|---|
| 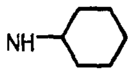 | 102 | | Fu(1.5) |
| 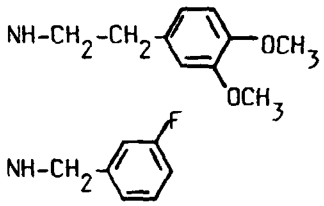 | 201 | | Fu |
| | 212 | | Cl |
| 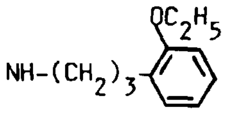 | 145 | | Fu(1.5) |
| 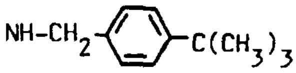 | 145 | | Fu(1.5) |
| 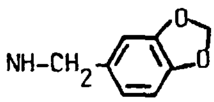 | 192 | | Fu(1.5) |
| 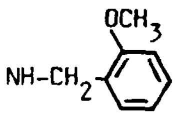 | 158 | | Fu(2) |
| 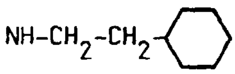 | 209 | | Cl |
| 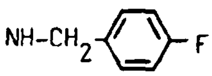 | 170 | | Cl |
| 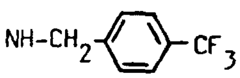 | 138 | | Cl |
| 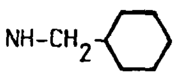 | 188 | | Cl |
| 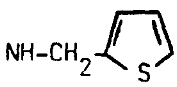 | 222 | | Cl |

| | Fp.(°C) | tautomere Struktur | Salz-form |
|---|---|---|---|
| 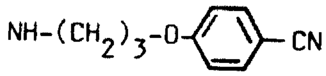 | 144 | | Fu(1.5) |
| 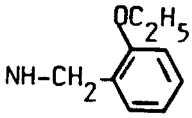 | 180 | | Fu(1.5) |
| 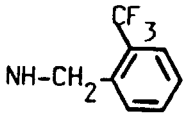 | 210 | | Fu(1.5) |
| 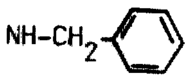 | 222 | | Cl |

Tabelle 2

| 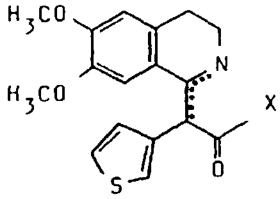 | Salzform | Fp.(°C) |
|---|---|---|
| X = 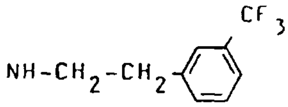 | OX | 125-135 (Zers.) |
| 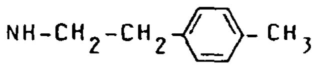 | BS | 161-163 |
| 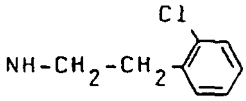 | | |
| 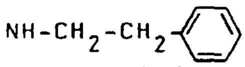 | | |
| 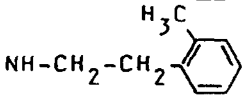 | BS | 118-120 |
| 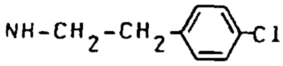 | | |
| 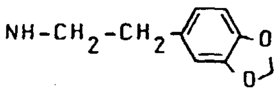 | | |
| 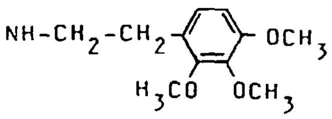 | | |
| 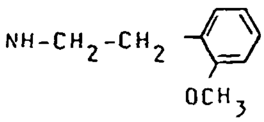 | | |
| 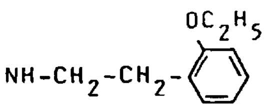 | | |
| 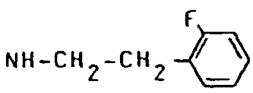 | BS | 128-130 |
| 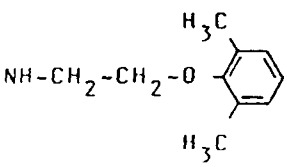 | | |

| | Salzform | Fp.(°C) |
|---|---|---|
| 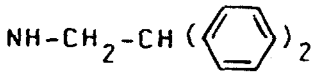 | BS | 185-187 |
| 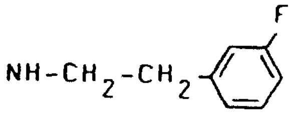 | | |
| 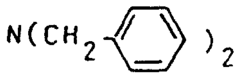 | OX | 128-130 |
| 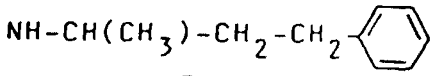 | | |
| 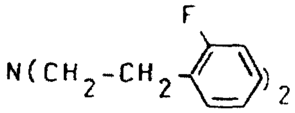 | | |
| 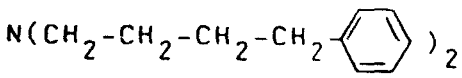 | | |
| 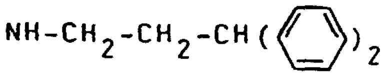 | OX | 70-80 (Zers.) |
| 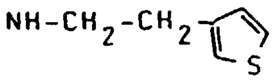 | BS | 165-167 |
| 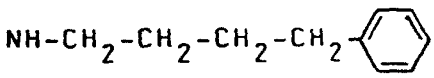 | BS | 102-104 |
| 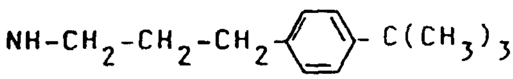 | OX | 124-127 |
| $NH-(CH_2)_9-CH_3$ | OX | 121-122 |
| 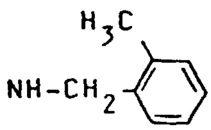 | | |

| | Salzform | Fp.(°C) |
|---|---|---|
| 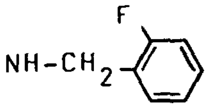 | | |
| 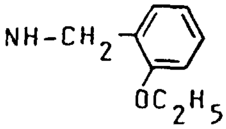 | | |
| 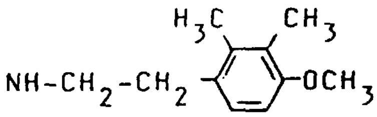 | | |
| 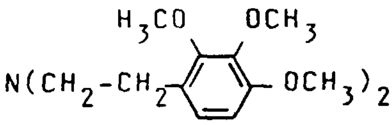 | OX | 107-112 |
| 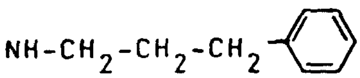 | | |
| $NH_2$ | OX | 121-140 (Zers.) |
| 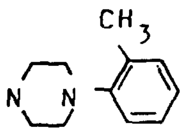 | OX | amorph |
| 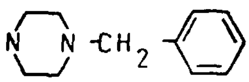 | OX | 80-100 (Zers.) |
| 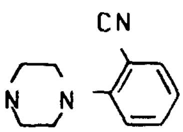 | | |

Tabelle 3

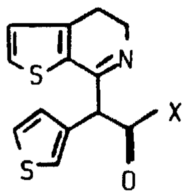

| X: | Salzform | Fp. (°C) |
|---|---|---|
| 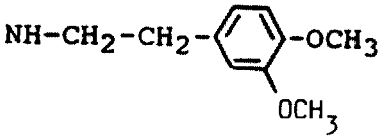 | BS | amorph |

| | |
|---|---|
| OX: | Oxalat |
| BS: | freie Base |
| Zers.: | Zersetzung |

Gegenstand der vorliegenden Erfindung ist ferner die Verwendung dieser neuen Verbindungen.

Die Verbindungen sind zur Behandlung von degenerativen und nekrotisierenden Erkrankungen des Gehirns vorteilhaft. Ebenso ist die vorbeugende Behandlung von durch solche Krankheiten gefährdeten Patienten möglich. Diese Wirkung der Verbindungen beruht nicht auf einer Verbesserung der Gewebsdurchblutung. Die Verbindungen sind somit für eine neuartige Behandlung von Epilepsie und der Alzheimer-Krankheit geeignet, und insbesondere bei der Behandlung von Patienten, die einen Schlaganfall erlitten haben oder gefährdet sind, einen Schlaganfall zu erleiden.

Ein weiterer Gegenstand der vorliegenden Erfindung ist die Verwendung der genannten Verbindungen für die Herstellung von Mitteln zur Behandlung chronisch inflammatorischer Prozesse, der Colitis Ulcerosa und des Morbus Crohn und von Mitteln mit antiproliferativer Wirkung. Die Wirkung der Verbindungen kann durch ihre Hemmung der unselektiven Kationenkanäle (UKK) erklärt werden.

Der Pathophysiologie des chronischen Bronchialasthma liegen entzündliche Prozesse zugrunde, die durch die Aktivierung inflammatorischer Zellen vermittelt werden. (BARNES, 1987; SEIFERT und SCHULTZ, 1991).

Die rezeptor-regulierte Aktivierung inflammatorischer Zellen (z.B. neutrophile Granulozyten und Mastzellen bzw. deren permanente Zellinien HL-60 Zellen oder sensibilisierte, d.h. Gammaglobulin E beladene RBL-Zellen) wird unabhängig von der Art der stimulierenden Agonisten (z.B. Endothelin, PAF, Leukotriene, chemotaktisches Peptid fMLP oder Antigen gegen sensibilisierte Mastzellen) durch Blocker unselektiver Kationenkanäle (UKK) inhibiert (RINK, 1990). Durch diese Kanäle gelangt extrazelluläres Calcium in die Zellen, das für die Persistenz der rezeptor-vermittelten Zellaktivierungen erforderlich ist (PUTNEY, 1990). Wird diese Calciumzufuhr unterbrochen resultiert eine Blockade der Aktivierung inflammatorischer Zellen.

Klassische Calciumantagonisten vom Dihydropyridin- bzw. Phenylalkylamin-Typ hemmen weder UKKs noch inflammatorische Prozesse (WELLS et al., 1986).

Als Maß der Zellaktivierung bzw. als Maß von deren Hemmung durch UKK-Blocker wird fluorometrisch die Kinetik der cytoplasmatischen Calciumionenkonzentration in Fura-2-beladenen Zellen anhand der von GRYNKIEWICZ et al. (1985) beschriebenen Methode quantifiziert. Diese Vorgehensweise hat sich im Rahmen dieser Erfindung als

zuverlässige Screeningmethode zur Auffindung von UKK-Blockern erwiesen.

Zur spezifischen Charakterisierung von Blockern der unselektiven Kationenkanäle eignet sich die sogenannte funktionelle THAPSIGARGIN-Inhibition. THAPSIGARGIN ist ein von THASTRUP et al. (Proc. Natl. Acad. Sci. (USA), 87, 2466-2470, 1990) beschriebener Tumorpromotor, der selektiv und irreversibel die $Ca^{2+}$ATPase intrazellulärer, $IP_3$-sensitiver $Ca^{2+}$-Speicher hemmt. Infolge dessen kommt es zu einer raschen Entleerung der $Ca^{2+}$-Speicher. Wie von J. PUTNEY (Calcium, 11, 611-624, 1990) beschrieben stellt die Entleerung dieser Speicher den physiologischen Reiz für die Öffnung unselektiver Kationenkanäle in der Zellmembran dar. Die Folge ist ein massiver Einstrom von $Na^+$ und $Ca^{2+}$ in die Zelle. Aufgrund dieser Eigenschaften eignet sich Thapsigargin als indirekter Stimulator zur agonisten- und $IP_3$-unabhängigen Öffnung unselektiver Kationenkanäle.

Im Rahmen der vorliegenden Erfindung wurde die Thapsigargin-Stimulation unselektiver Kationenkanäle an HL 60 Zellen (menschliche Leukämiezelle), an hippocampalen und corticalen Neuronenzellen sowie an RBL-Zellen (rat basophilic lymphoma cells) erfolgreich durchgeführt und somit wurde die Existenz dieser Kanäle in den jeweiligen Zellinien nachgewiesen.

Die zytoplasmatische $Ca^{2+}$Konzentration ($[Ca^{2+}]_i$) spielt eine wichtige Rolle bei der Zellproliferation und beim Tumorwachstum (Übersicht siehe L.R. ZACHARSKI, Journal of Medicine 19: 145-177, 1988). Insbesondere der durch Rezeptoraktivierung mit konsekutiver Inositoltrisphosphat-($IP_3$-)-Vermittlung stimulierte $Ca^{2+}$-Einstrom in die Zelle soll von entscheidender Bedeutung sein für die oncogene Zellproliferation (U. KIKKAWA und Y. NISHIZUKA, Ann. REV. CELL. BIOL. 2: 149-178, 1986). Dieser Mechanismus spielt auch eine Rolle bei der Metastasenbildung und der "Multi-Drug-Resistance". (Übersicht siehe die obengenannten Veröffentlichung von L.R. ZACHARSKI.) J. MED. 19: 145-177, 1980.

Diese Hypothese wird dadurch gestützt, daß Thapsigargin als indirekter Stimulator der unselektiven Kationenkanäle (UKK) sowohl zu einem $Ca^{2+}$-overload in der Zelle führt als auch ein hochwirksamer Tumorpromotor ist.(V. THASTRUP et al. Proceedings of the NATL. Acad. Sci: (USA) 87: 2466-2470, 1990.)

Die Blockade des $Ca^{2+}$-Einstroms durch die UKK führt zu einer Normalisierung der intrazellulären Ca-Ionenkonzentration und somit zu einer Hemmung des Tumorwachstums etc.

Klassiche Calciumantagonisten hemmen diese UKK nicht. Überraschend ist festgestellt worden, daß die Verbindungen dieser Erfindung den Calciumeinstrom in die Zelle durch die UKK hemmen.

Wie von S. H. MURCH et al. (Lancet 339 : 381-385, 15. Febr. 1992) gezeigt wurde, spielt Endothelin I eine wichtige pathophysiologische Rolle bei entzündlichen Darmerkrankungen wie der Colitis ulcerosa und des Morbus Crohn. Mit Hilfe immunhistochemischer Methoden konnte festgestellt werden, daß Patienten mit M. Crohn im Bereich der Submucosa und Patienten mit Colitis ulcerosa im Bereich der Lamina propria des Dickdarmepithels signifikant und stark erhöhte Endothelin I Konzentrationen im Vergleich zu gesunden Normalpersonen aufweisen. Es wird angenommen, daß die lokale Ausschüttung von Endothelin massive Vasospasmen mit konsekutiver disseminierter Ischämie mit Mikroinfarkten hervorruft, die als eigentliche Ursache der genannten Erkrankungen angesehen werden. Die vasospasmogene Effektivität des Endothelins wird über einen $Ca^{2+}$-overload vaskulärer Myozyten erklärt. Dabei löst Endothelin primär eine $IP_3$-vermittelte intrazelluläre $Ca^{2+}$-Freisetzung aus, an die sich ein massiver transmembranärer $Ca^{2+}$-Einstrom durch Dihydropyridin-insensitive Kanäle anschließt. (M. S. Simonson et al. Clin. Invest. Med. 14: 499-507, 1991; T. Masakai, J. Cardiovasc. Pharmacol. 13:Suppl. 5, S1-S4, 1989; D. W. Hay, R. J. Pharmacol. 100: 383-392, 1990) Bei diesen Kanälen handelt es sich um unselektive Kationen Kanäle, deren Existenz kürzlich auch in Zellen der Dickdarmmukosa beschrieben wurde. (Chr. Siemer und H. Gögelein, Europ. J. Physiol. 420: 319-328, 1992).

Als geeignetes Screening Modell zur Auffindung funktioneller Endothelinantagonisten hat sich die Endothelin stimulierte Aktivierung Fura-2 beladener menschlicher Leukämiezellen (HL 60 Zelle) bewährt. In Anlehnung an G. GRYNKIEWICZ et al. (J. Biol. Chem. 260:3440-3450, 1985)läßt sich die intrazelluläre $Ca^{2+}$-Konzentration im Cytoplasma von HL 60 Zellen (Suspensionen) spektrofluorometrisch verfolgen und als Maß der Zellaktivierung durch Endothelin quantifizieren. Die Stimulation erfolgte durch Zusatz von 0.1 mM Endothelin, sie ließ sich dosisabhängig durch die erfindungsgemäßen Substanzen inhibieren.

Der funktionelle Endothelinantagonismus der erfindungsgemäßen Substanzen wird über eine Blockade unselektiver Kationen Kanäle vermittelt. Deshalb eignet sich auch der Nachweis eines funktionellen Thapsigargin-Antagonismus an RBL-hm1-Zellen als Screening Methode für funktionelle Endothelinantagonisten.

Ausführung der Untersuchung:

Für Screeningzwecke werden in $Ca^{2+}$ freiem Inkubationsmedium Fura-2-beladene adhäsive RBL-hm 1-Zellen mit 0,1 μM Thapsigargin stimuliert. Nach 4 Minuten wird extrazelluläres $Ca^{2+}$ auf 1,5 mM restituiert und anhand der Fura-2-Fluoreszenz der exzessive Anstieg der cytoplasmatischen $Ca^{2+}$-Konzentration infolge eines massiven transmembranären $Ca^{2+}$-Einstroms durch unselektive Kationenkanäle registriert.

Dieser Einstrom ist ausschließlich und dosisabhängig zu inhibieren durch unselektive Kationen-Kanal- Blokker. Weder klassische Kalziumantagonisten noch spezifische Blocker von Agonisten, die den $IP_3$- Turnover stimulieren

können den durch Thapsigargin indirekt ausgelösten transmembranären $Ca^{2+}$-Einstrom hemmen. Die Verbindungen der vorliegenden Erfindung zeichnen sich durch eine Hemmung der UKK aus.

Die fluorometrische Calciummessung im Cytoplasma einzelner adhärenter RBL-hm1-Zellen erfolgt in Analogie zu der von KUDO und OGURA. (1986) für neuronale Zellen beschriebenen Methode. Verwendet wird ein AXIOVERT 35 Fluoreszenzmikroskop von ZEISS in Kombination mit einem Imaging-System von HAMAMATSU, bestehend aus dem ICMS-Bildverarbeitungssystem, Restlichtkamera mit Kontrolleinheit und Bildverstärker DVS 3000.

Die Kinetik der cytoplasmatischen $Ca^{+2}$-Konzentration wird als Konzentrations-Zeit-Kurve nach der durch Thapsigargin (0,1 µM) stimulierten Zellaktivierung fortlaufend aufgezeichnet. Verglichen werden die Kurven zweier aktivierter Zellkulturen in Gegenwart und Abwesenheit von 10 µM Testsubstanz. Die Fläche unter diesen Kurven (area under the curve = AUC) wird integriert und als Maß der Zellaktivierung registriert. Die inhibitorische Wirkungsstärke der getesteten UKK-Blocker wird ermittelt anhand folgender Beziehung:

$$\%H = 100 - \frac{AUC_{inh} x100}{AUC_{(Kontrolle)}}$$

%H = die prozentuale Hemmung des Calciumeinstroms durch unselektive Kationenkanäle der stimuliert und durch 10 µM Testsubstanz inhibiert wird.

$AUC_{inh}$ = Fläche unter der Kurve, die in Gegenwart des Stimulans plus 10 µM inhibitorischer Testsubstanz aufgezeichnet wird.

AUC Kontr. = Fläche unter der Kurve, die nur nach Zusatz des Stimulans registriert wird.

Literatur zu den obigen Erläuterungen:


BARNES P.J., I.W. RODGER und N.C. THOMSON
Pathogenesis of asthma, in "ASTHMA, basic mechanisms and clinical management"
ED by P.J. BARNES; ACADEMIC PRESS, LONDON, 1988

GRYNKIEWICZ G., M. POENIE und R.Y. TSIEN
A new generation of $Ca^{2+}$-indicators with greatly improved fluorescence properties
J. BIOL. CHEM. 260: 3440-3450, 1985

HIDE, M. und M.A. BEAVEN
Calcium influx in a rat mast cell (RBL-2H3) line
J. BIOL. CHEM. 266 15221-15229, 1991

KUDO, Y. und A. OGURA
Glutamate-induced increase in intracellular $Ca^{2+}$-concentration in isolated hippocampal neurones
BR. J. PHARMACOL. 89: 191-198, 1986

PUTNEY, J.W., jr.
Capacitative Calcium entry revised
CELL CALCIUM 11: 611-624, 1990

RINK, T.J.
Receptor-mediated calcium entry
FEBS LETT. 268: 381-385, 1990

SEIFERT, R. und G. SCHULTZ
The superoxide forming NADPH oxidase of phagocytes: An enzym system regulated by multiple mechanism
REV. PHYSIOL. BIOCHEM. PHARMACOL., Vol. 117,
SPRINGER VERL., 1991

WELLS, E., C.G. JACKSON, S.T. HARPER, J. MANN and R.P. EAOY
Characterization of primate bronchoalveolar mast cells II, inhibition of histamine, $LTC_4$ and $PGF_{2a}$ release from primate bronchoalveolar mast cells and a comparison with rat peritoneal mast cells

J. IMMUNOL. 137: 3941-3945, 1986.

Meßergebnisse:

Angegeben wird die prozentuale Hemmung der UKK nach Thapsigarginstimulation (0,1 µM Thapsigargin) in RBL-hm 1 Zellen. Die einheitliche Konzentration der Testsubstanzen ist $10^{-5}$ Mol beziehungsweise $10^{-6}$ Mol).

Tabelle 4

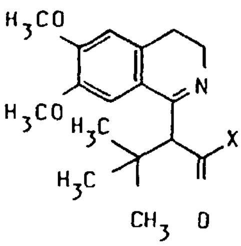

| | Salz-form | %H($10^{-5}$ M) | %H($10^{-6}$ M) |
|---|---|---|---|
| X = 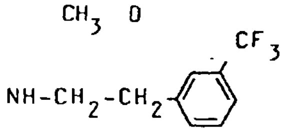 | Fu(1.5) | | 71.9 |
| 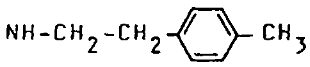 | Fu | | 57.3 |
| 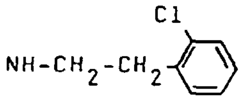 | Fu(1.5) | | 71.2 |
| 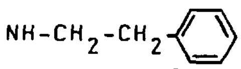 | Cl | | 66.4 |
| 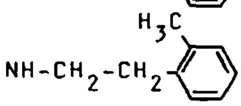 | Fu(1.5) | | 53.3 |
| 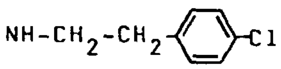 | Fu | | 70.7 |
| 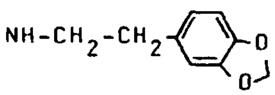 | | | |
| 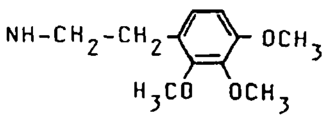 | | | |
| 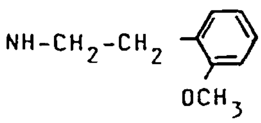 | Fu(1.5) | 60.5 | |
| 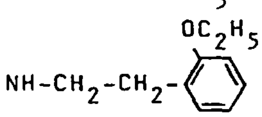 | | | |
| 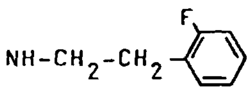 | Cl | | 64.6 |
| 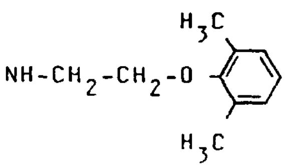 | | | |

| | Salz-form | %H($10^{-5}$M) | %H($10^{-6}$M) |
|---|---|---|---|
| 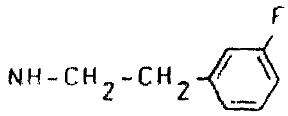 | Cl | | 70.1 |
| 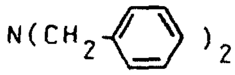 | | | |
| 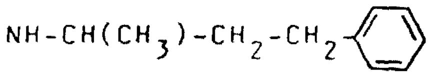 | | | |
| 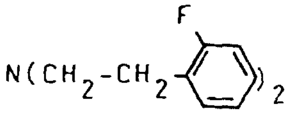 | | | |
| 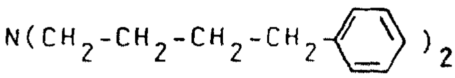 | | | |
| 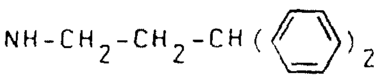 | Fu | | 77.2 |
| 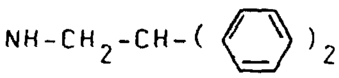 | Fu | | 75.7 |
| 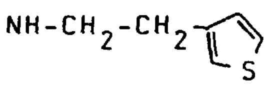 | Cl | | 24.8 |
| 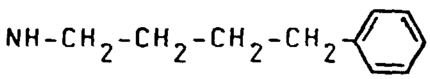 | Cl | | 64.4 |
| 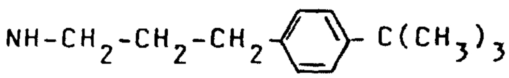 | Fu(1.5) | | 74.4 |
| $NH-(CH_2)_9-CH_3$ | Cl | | 69.7 |
| 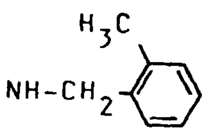 | Cl | | 45.8 |

| | Salz-form | %H($10^{-5}$M) | %H($10^{-6}$M) |
|---|---|---|---|
| 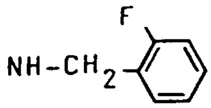 | Cl | | 80.8 |
| 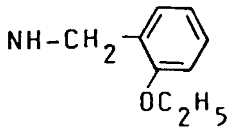 | | | |
| 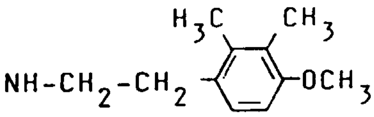 | | | |
| 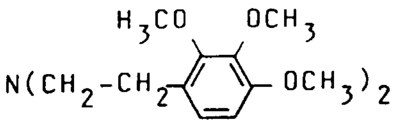 | | | |
| 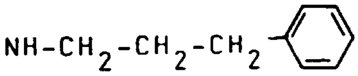 (3) | Cl | | 64.9 |
| 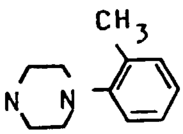 | Fu(1.5) | 66.8 | |
| 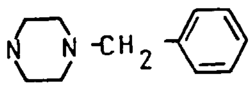 | | | |
| 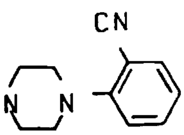 | | | |

| | Salz-form | %H($10^{-5}$M) | %H($10^{-6}$M) |
|---|---|---|---|
| 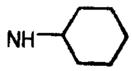 | Fu(1.5) | | 73.8 |
| 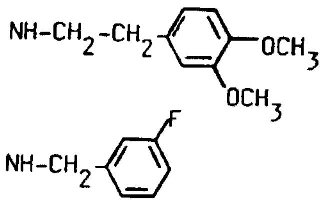 | Fu | | 29.2 |
| | Cl | | 72.7 |
| 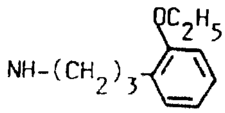 | Fu(1.5) | | 28.4 |
| 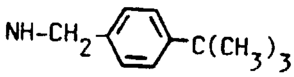 | Fu(1.5) | | 43.2 |
| 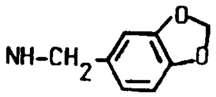 | Fu(1.5) | | 16.9 |
| 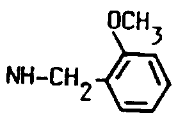 | Fu(2) | | 35.7 |
| 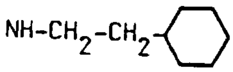 | Cl | | 53.3 |
| 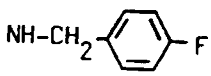 | Cl | 100.0 | 60.3 |
| 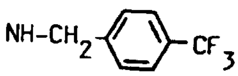 | Cl | 93.5 | 47.3 |
| 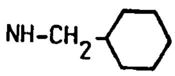 | Cl | 99.0 | 63.7 |
| 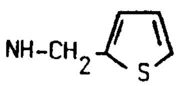 | Cl | 96.0 | 65.4 |

| | Salz-form | %H($10^{-5}$M) | %H($10^{-6}$M) |
|---|---|---|---|
| 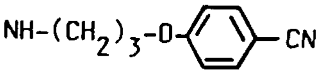 | Fu(1.5) | 91.6 | 65.7 |
| 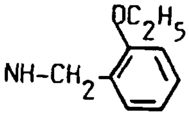 | Fu(1.5) | 65.8 | |
| 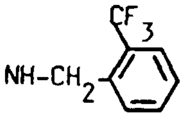 | Fu(1.5) | | 51.1 |
| 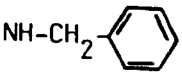 | Cl | | 61.0 |

Tabelle 5

| 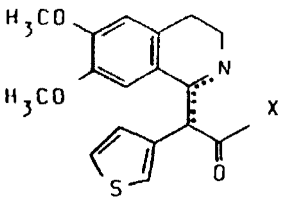 | Salz-form | %H($10^{-5}$M) |
|---|---|---|
| X = 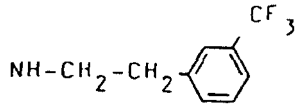 | OX | 20.85 |
| 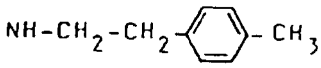 | BS | 59.94 |
| 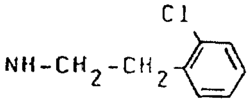 | | |
| 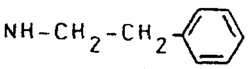 | | |
| 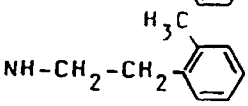 | BS | 37.83 |
| 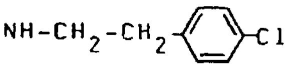 | | |
| 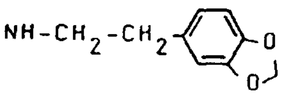 | | |
| 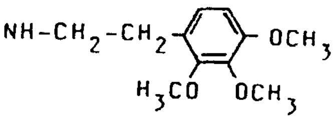 | | |
| 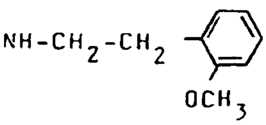 | | |
| 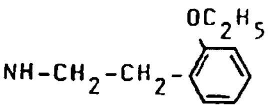 | | |
| 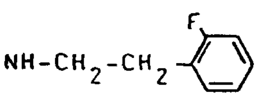 | BS | 36.41 |
| 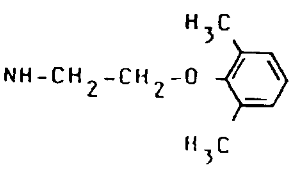 | | |

| | Salzform | %H($10^{-5}$M) |
|---|---|---|
| 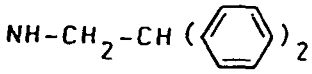 | BS | 39.17 |
| 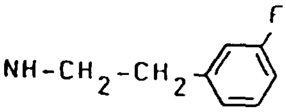 | | |
| 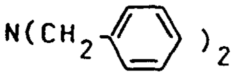 | OX | 90.9 |
| 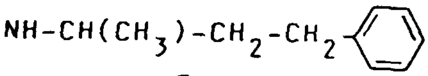 | | |
| 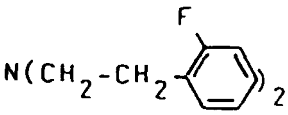 | | |
| 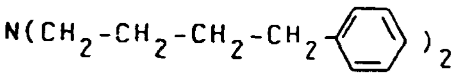 | | |
| 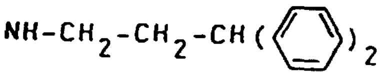 | OX | 22.62 |
| 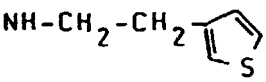 | BS | 14.6 |
| 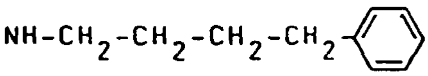 | BS | 25.76 |
| 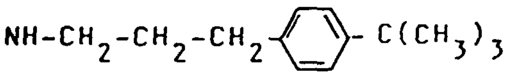 | OX | 50.61 |
| $NH-(CH_2)_9-CH_3$ | OX | 47.09 |
| 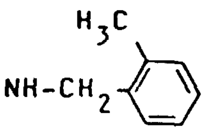 | | |

| | Salzform | %H($10^{-5}$M) |
|---|---|---|
| 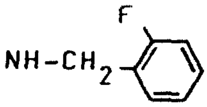 | | |
| 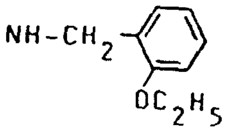 | | |
| 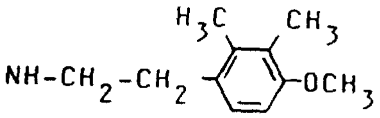 | | |
| 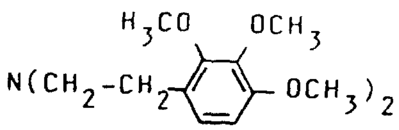 | OX | 28.14 |
| 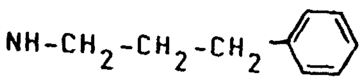 | | |
| $NH_2$ | OX | |
| 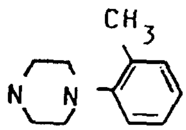 | OX | 6.8 |
| 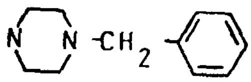 | OX | 52.26 |
| 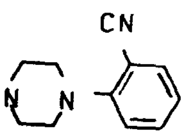 | | |

Tabelle 6

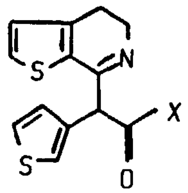

| | Salzform | %H($10^{-5}$M) |
|---|---|---|
| 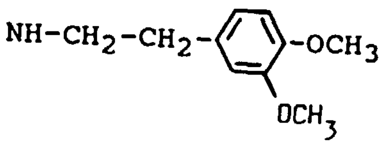 | BS | 27.01 |

Anhand des folgenden Tests kann die funktionelle antiinflammatorische Effektivität gezeigt werden:

Verwendet werden einzelne an Glasplättchen adhärente RBL-2H3-Zellen (eine den Mastzellen verwandte Tumorzellinie).

Die Kultivierung und Anhaftung der RBL-2H3-Zellen erfolgt in der HIDE und BEAVEN (1991) beschriebenen Methode. Zur Sensibilisierung der adhäsiven RBL-2H3-Zellen werden die Zellen 2 stunden bei Raumtemperatur mit einer 1:2000 verdünnten käuflichen Gammaglobulin E-Lösung gegen einen Dinitrophenol- Rinderserumalbumin-Komplex (DNP-BSA-Antigen) inkubiert. Anschließend werden die Zellen gewaschen. Durch Zusatz von 0,1 ml DNP-BSA-Lösung (10 µg/ml) erfolgt eine massive immunologische Zellaktivierung, die durch einen cytoplasmatischen $Ca^{2+}$-overload vermittelt wird. Die fluorometrische Calciummessung im Cytoplasma einzelner adhärenter RBL-2H3-Zellen erfolgt in Analogie zu der von KUDO und OGURA (1986) für neuronale Zellen beschriebenen Methode, die auch weiter oben in dieser Beschreibung erläutert wird.

Als Vergleichssubstanz dient in diesen Untersuchungen (10 µM) Chromoglycat, das eine ca. 50 %ige Hemmung der antigen-induzierten Zellaktivierung bewirkt.

In diesem Test zeigen die oben genannten Verbindungen %H Werte, die den oben angegebenen Werten vergleichbar sind.

In Untersuchungen an Mikrokulturen verschiedener menschlicher Tumorzellinien mit Hilfe des Tetrazolium Assays zur Feststellung des antiproliferativen Effektes der erfindungsgemäßen Substanzen zeigte sich überraschenderweise, daß die geprüfte Verbindung 5 bis 100 mal wirkungsstärker als die Vergleichssubstanz Verapamil sind.

Die antiproliferative Effektivität der Testsubstanzen wurde mit Hilfe des von MOSMANN (J. IMMUNOL. METH. 65: 55-63, 1983), DENIZOT et al. (J. IMMUNOL. METH. 89: 271-277, 1986) und von J. ELIASON et al. (INT. J. CANCER 46: 113-117, 1990) beschriebenen MTT-Tests bestimmt. (MTT = [3-(4,5-dimethylthiazol-2yl)2,5-diphenyltetrazoliumbromid] von CHEMICON Inc. El Segundo, Ca, USA). Dieser Indikator wird nur von lebenden Zellen mit intakten Mitochondrien zu einem blauen Formazan Produkt metabolisiert. Folgende menschliche Tumorzellinien wurden in unserem Test verwendet: A 549 (Adenocarcinom der Lunge), A 431 (Epidermiscarcinom der Vulva), PC 3 (Adenocarcinom der Prostata), SK BR 3 (Adenocarcinom der Mamma), HT 29 (CX1 1) (Adenocarcinom des Colon) und K 562 (chronisch-myeloische Leukämiezelle).

Der Test wurde auf Mikrotiterplatten durchgeführt. Jedes Well enthielt 100 µl einer Zellsuspension (0,2 x $10^6$ Zellen/ml). Als Inkubationsmedium diente RPMI 1640 mit 10% Hitze-inaktiviertem fetalen Kälberserum und 50 µg/ml Gentamycin. Die Zellsuspensionen wurden 0, 24, 48 oder 72 Stunden bei gesättigter Luftfeuchtigkeit in einem $CO_2$ (5%) - Luft (95%)-Gemisch bei 37°C inkubiert in Gegenwart und Abwesenheit von variablen Konzentrationen antiproliferativer Testsubstanzen. Die Testsubstanzen wurden in DMSO (Endverdünnung: 0,1 %) gelöst. Anschließend wurden 10 µl MTT-Lösung (3 mg/ml) und nach 3 Stunden 100 µl einer 0,08 N HCl enthaltenden Isopropanollösung zugesetzt. Nach einer weiteren Stunde wurde die Lichtabsorption bei 570 nm (Vergleichswellenlänge 630 nm) in einem Mikroplattenleser ermittelt. Die Lichtabsorption ist direkt proportional zur Anzahl lebender Zellen. Die halbmaximalen Hemmkonzentrationen der untersuchten Substanzen lagen bei 1 µg/ml.

Die vasospasmolytische Effektivität der obengenannten funktionellen Endothelin- bzw. Thapsigargin-Antagonisten wurde am isolierten Gefäßpräparat bestätigt: An retrograd perfundierten spontan schlagenden LANGENDORFF Herzen aus Ratten wurde die coronare Perfusion fortlaufend mittels elektromagnetischer Flußmessung (Apparatur von Hugo Sachs Elektronik, MARCH), quantifiziert. Mit dieser Meßanordnung lassen sich Ausmaß, Dauer und Verlaufsform von Gefäßspasmen mit großer Genauigkeit registrieren. Perfundiert man mit 100 nM Endothelinkonzentration, so wird der coronare Perfusionsfluß von 11 auf 5 ml/min reduziert. Die Perfusionseinschränkung kann durch die erfindungsgemäßen Substanzen aufgehoben werden. Die Wirkungsstärken der erfindungsgemäßen Verbindungen bezüglich der Thapsigargininhibition an Fura-2- beladenen RBL-hm1-Zellen bzw. der Effektivität der Endothelininhibition an Fura-2 beladenen HL 60 Zellen korrelierte eindeutig mit der am Langendorffpräparat nachgewiesenen vasospasmolytischen Effektivität der untersuchten Substanzen. Es kann daraus geschlossen werden, daß dem vasospasmolytischen Endothelinantagonismus der untersuchten Substanzen eine Blockade unselektiver Kationen Kanäle unterliegt.

Pharmazeutische Anwendungsbeispiele

| a) Dragees<br>1 Drageekern enthält: | |
|---|---|
| Wirkstoff der allgemeinen Formel I | 30,0 mg |
| Milchzucker | 100,0 mg |
| Maisstärke | 75,0 mg |
| Gelatine | 3,0 mg |
| Magnesiumstearat | 2,0 mg |
| | 210,0 mg |

Herstellung

Die Mischung der Wirksubstanz mit Milchzucker und Maisstärke wird mit einer 10%-igen wässerigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässerigen Suspension von Zucker, Titandioxyd, Talkum und Gummi arabicum aufgebracht wird. Die fertigen Dragees werden mit Bienenwachs poliert.

| b) Tabletten | |
|---|---|
| Wirkstoff der allgemeinen Formel I | 30,0 mg |
| Milchzucker | 100,0 mg |
| Maisstärke | 70,0 mg |
| löslich Stärke | 7,0 mg |
| Magnesiumstearat | 3,0 mg |
| | 210,0 mg |

Herstellung

Wirkstoff und Magnesiumstearat werden mit einer wässerigen Lösung der löslichen Stärke granuliert, das

Granulat getrocknet und innig mit Milchzucker und Maisstärke vermischt. Das Gemisch wird sodann zu Tabletten von 210 mg Gewicht verpreßt.

| c) Kapseln | |
|---|---|
| Wirkstoff gemäß Formel I | 20,0 mg |
| Milchzucker | 230,0 mg |
| Maisstärke | 40,0 mg |
| Talk | 10,0 mg |
| | 300,0 mg |

Herstellung

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

| d) Tabletten | |
|---|---|
| Wirkstoff gemäß Erfindung | 40,0 mg |
| Milchzucker | 100,0 mg |
| Maisstärke | 50,0 mg |
| kolloidale Kieselsäure | 2,0 mg |
| Magnesiumstearat | 3.0 mg |
| insgesamt | 195,0 mg |

Herstellung:

Der Wirkstoff wird mit einem Teil der Hilfsstoffe gemischt und mit einer Lösung der löslichen Stärke in Wasser granuliert. Nach dem Trocknen des Granulats wird der Rest der Hilfsstoffe zugemischt und die Mischung zu Tabletten verpreßt.

| e) Dragées | |
|---|---|
| Wirkstoff gemäß Erfindung | 20,0 mg |
| Milchzucker | 100,0 mg |
| Maisstärke | 65,0 mg |
| kolloidale Kieselsäure | 2,0 mg |
| lösliche Stärke | 5,0 mg |
| Magnesiumstearat | 3,0 mg |
| insgesamt | 195,0 mg |

Herstellung:

Der Wirkstoff und die Hilfsstoffe werden, wie in Beispiel a beschrieben, zu Tablettenkernen verpreßt, die mit Zucker, Talcum und Gummi arabicum in üblicher Weise dragiert werden.

| f) Suppositorien | |
|---|---|
| Wirkstoff gemäß Erfindung | 50,0 mg |
| Milchzucker | 250,0 mg |
| Suppositorienmasse q.s. ad | 1,7 g |

Herstellung:

Der Wirkstoff und der Milchzucker werden miteinander vermischt und die Mischung in der geschmolzenen Suppositorienmasse gleichmäßig suspendiert. Die Suspensionen werden in gekühlte Formen zu Suppositorien von 1,7 g Gewicht ausgegossen.

| g) Ampullen | |
|---|---|
| Wirkstoff gemäß Erfindung | 20,0 mg |
| Natriumchlorid | 5,0 mg |
| Bi-destilliertes Wasser q.s. ad | 2,0 ml |

Herstellung:

Der Wirkstoff und das Natriumchlorid werden in bi-destilliertem Wasser gelöst und die Lösung in Ampullen steril abgefüllt.

| h) Ampullen | |
|---|---|
| Wirkstoff gemäß Erfindung | 10,0 mg |
| Natriumchlorid | 7,0 mg |
| Bi-destilliertes Wasser q.s. ad | 1,0 ml |

| i) Tropfen | |
|---|---|
| Wirkstoff gemäß Erfindung | 0,70 g |
| p-Hydroxybenzoesäuremethylester | 0,07 g |
| p-Hydroxybenzoesäurepropylester | 0,03 g |
| Entmineralisiertes Wasser q.s. ad | 100,00 ml |

Herstellung:

Der Wirkstoff und die Konservierungsmittel werden in entmineralsiertem Wasser gelöst, die Lösung filtriert und in Flaschen zu je 100 ml abgefüllt.

## Patentansprüche

**1.** Verbindung der allgemeinen Formel I

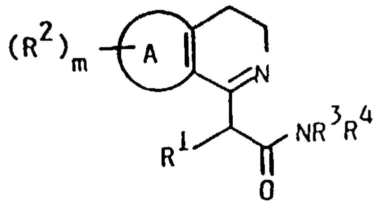

I

worin

A einen Benzo, Indolo oder Thienorest bedeutet;
worin, wenn A Benzo ist, m 2 oder 3 ist,

und die Substituenten $R^2$ unabhängig voneinander Hydroxy, $(C_1-C_4)$Alkoxy, Benzyloxy, Halogen (F, Cl, Br, J), $(C_1-C_4)$Alkyl, Methansulfonyloxy oder Methansulfonamido, oder zwei benachbarte Substituenten $R^2$ zusammen -0-$CH_2$-0- oder -0-$CH_2$-$CH_2$-O- sein können; und wenn A Indolo oder Thieno ist, m Null ist;

$R^1$ Thienyl oder die Gruppe

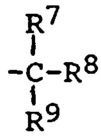

bedeutet,
worin

$R^7$, $R^8$ und $R^9$ unabhängig voneinander Methyl, Ethyl, Propyl, Phenyl oder Benzyl sein können, wobei nicht mehr als 2 der Substituenten gleichzeitig Phenyl oder Benzyl sein können;

$R^3$ und $R^4$ unabhängig voneinander

(a) Wasserstoff,
(b) verzweigtes oder unverzweigtes Alkenyl mit 3 bis 6 Kohlenstoffatomen,
(c) verzweigtes oder unverzweigtes Alkinyl mit 3 bis 6 Kohlenstoffatomen, oder
(d) verzweigtes oder unverzweigtes Alkyl mit 1-12 Kohlenstoffatomen bedeuten, wobei das Alkyl substituiert sein kann durch

Hydroxy,
$(C_1-C_4)$Alkoxy,
Di$(C_1-C_4)$Alkylamino,
Furyl,
Pyridyl,

Pyrrolidinyl, N-Methylpyrrolidinyl,
Morpholino,
Indolyl,
Nitrilo,
Thienyl,
Adamantyl,
Cyclohexyl,
Phenoxy,
Naphthyloxy oder Phenyl [wobei dieses Phenyl oder das in der Phenoxygruppe enthaltene Phenyl ein-, zwei- oder dreifach durch Hydroxy, $(C_1-C_4)$Alkoxy, Benzyloxy, Halogen (F, Cl, Br, J), $CF_3$, $N_3$, CN, $(C_1-C_4)$Alkyl, Adamantyl, $-SO_2NH_2$, $-NHCOCH_3$, $-NHSO_2CH_3$ oder $CH_3SO_2O$- oder durch die Brücke $-O-CH_2-O-$ substituiert sein kann] oder durch zwei unsubstituierte Phenylgruppen;
oder $R^3$ Wasserstoff bedeutet und $R^4$ Cyclohexyl, Phenyl [wobei dieses Phenyl ein-, zwei- oder dreifach durch Hydroxy, $(C_1-C_4)$Alkoxy, Benzyloxy, Halogen (F, Cl, Br, J), $CF_3$, $N_3$, $(C_1-C_4)$Alkyl, Adamantyl, $-SO_2NH_2$, $-NHCOCH_3$, $-NHSO_2CH_3$ oder $CH_3SO_2O$- oder durch die Brücke $-O-CH_2-O-$substituiert sein kann], Pyridyl oder N-Benzylpiperidyl;
oder $R^3$ und $R^4$ gemeinsam mit dem Stickstoffatom, an das sie gebunden sind,
Pyrrolidinyl,
Piperidinyl,
Morpholinyl, Thiomorpholinyl
oder
Piperazinyl bedeuten, wobei der Piperazinylring gegebenenfalls durch Methyl, unsubstituiertes Phenyl, Mono- oder Di$(C_1-C_4)$alkoxyphenyl, Cyano substituiertes Phenyl, Pyrimidinyl, Phenyl$(C_1-C_4)$alkyl, $(C_1-C_4)$Alkylphenyl oder

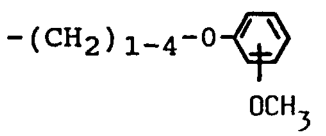

N-substituiert sein kann;

oder deren Salze mit physiologisch verträglichen Säuren oder Komplexbildnern.

**2.** Verbindung nach Anspruch 1, worin

$R^3$ und $R^4$ unabhängig voneinander

(a) Wasserstoff,
(b) verzweigtes oder unverzweigtes Alkenyl mit 3 bis 6 Kohlenstoffatomen,
(c) verzweigtes oder unverzweigtes Alkinyl mit 3 bis 6 Kohlenstoffatomen, oder
(d) verzweigtes oder unverzweigtes Alkyl mit 1-12 Kohlenstoffatomen bedeuten, wobei das Alkyl substituiert sein kann durch

Hydroxy,
$(C_1-C_4)$Alkoxy,
Di$(C_1-C_4)$Alkylamino,
Furyl,
Pyridyl,
Pyrrolidinyl, N-Methylpyrrolidinyl,
Morpholino,
Indolyl,
Nitrilo,
Thienyl,
Adamantyl,

Cyclohexyl,

Phenoxy,

Naphthyloxy oder Phenyl [wobei dieses Phenyl oder das in der Phenoxygruppe enthaltene Phenyl ein-, zwei- oder dreifach durch Hydroxy, $(C_1\text{-}C_4)$Alkoxy, Benzyloxy, Halogen (F, Cl, Br, J), $CF_3$, $N_3$, $(C_1\text{-}C_4)$Alkyl, Adamantyl, $-SO_2NH_2$, $-NHCOCH_3$, $-NHSO_2CH_3$ oder $CH_3SO_2O$- oder durch die Brücke $-O-CH_2-O-$ substituiert sein kann]

oder $R^3$ Wasserstoff bedeutet und $R^4$ Cyclohexyl, Phenyl [wobei dieses Phenyl ein-, zwei- oder dreifach durch Hydroxy, $(C_1\text{-}C_4)$Alkoxy, Benzyloxy, Halogen (F, Cl, Br, J), $CF_3$, $N_3$, $(C_1\text{-}C_4)$Alkyl, Adamantyl, $-SO_2NH_2$, $-NHCOCH_3$, $-NHSO_2CH_3$ oder $CH_3SO_2O$- oder durch die Brücke $-O-CH_2-O-$substituiert sein kann], Pyridyl oder N-Benzylpiperidyl;

oder $R^3$ und $R^4$ gemeinsam mit dem Stickstoffatom, an das sie gebunden sind,

Pyrrolidinyl,

Piperidinyl,

Morpholinyl, Thiomorpholinyl

oder

Piperazinyl bedeuten, wobei der Piperazinylring gegebenenfalls durch Methyl, unsubstituiertes Phenyl, Mono- oder Di$(C_1\text{-}C_4)$alkoxyphenyl, Pyrimidinyl, Phenyl$(C_1\text{-}C_4)$alkyl, oder

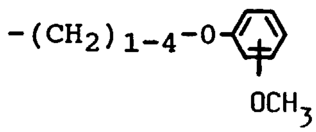

N-substituiert sein kann;

oder deren Salze mit physiologisch verträglichen Säuren oder Komplexbildnern.

**3.** Verbindung der allgemeinen Formel I nach Anspruch 1 oder 2 worin

A einen Benzo- oder Thienorest bedeutet;

worin, wenn A Benzo ist, m 2 ist, die beiden $R^2$ in den Positionen 6 und 7 sind und unabhängig voneinander Hydroxy, $(C_1\text{-}C_4)$Alkoxy, Benzyloxy, Halogen (F, Cl, Br, J), $(C_1\text{-}C_4)$Alkyl, Methansulfonyloxy oder Methansulfonamido, oder zwei benachbarte Substituenten

$R^2$ zusammen $-O-CH_2-O-$ oder $-O-CH_2-CH_2-O-$ sein können; und wenn A Thieno ist, m Null ist;

$R_1$ wie in Anspruch 1 definiert ist,

$R^3$ und $R^4$ unabhängig voneinander

(a) Wasserstoff,

(b) verzweigtes oder unverzweigtes Alkenyl mit 3 bis 6 Kohlenstoffatomen,

(c) verzweigtes oder unverzweigtes Alkinyl mit 3 bis 6 Kohlenstoffatomen, oder

(d) verzweigtes oder unverzweigtes Alkyl mit 1-12 Kohlenstoffatomen bedeuten, wobei das Alkyl substituiert sein kann durch

Hydroxy,

$(C_1\text{-}C_4)$Alkoxy,

Di$(C_1\text{-}C_4)$Alkylamino,

Furyl,

Pyridyl,

Pyrrolidinyl, N-Methylpyrrolidinyl,

Morpholino,

Indolyl,

Nitrilo,
Thienyl,
Adamantyl,
Cyclohexyl,
Phenoxy,
Naphthyloxy oder Phenyl, wobei dieses Phenyl oder das in der Phenoxygruppe enthaltene Phenyl ein-, zwei- oder dreifach durch Hydroxy, $(C_1-C_4)$Alkoxy, Benzyloxy, Halogen (F, Cl, Br, J), $CF_3$, $N_3$, $(C_1-C_4)$Alkyl, Adamantyl, $-SO_2NH_2$ oder $-NHCOCH_3$, oder durch die Brücke $-O-CH_2-O-$ substituiert sein kann;
oder $R^3$ Wasserstoff bedeutet und $R^4$ Cyclohexyl, Phenyl, Fluorophenyl, Pyridyl oder N-Benzylpiperidyl;
oder $R^3$ und $R^4$ gemeinsam mit dem Stickstoffatom, an das sie gebunden sind,
Pyrrolidinyl,
Piperidinyl,
Morpholinyl, Thiomorpholinyl
oder
Piperazinyl bedeuten, wobei der Piperazinylring gegebenenfalls durch Methyl, unsubstituiertes Phenyl, Mono- oder Di$(C_1-C_4)$alkoxyphenyl, Pyrimidinyl, Phenyl$(C_1-C_4)$alkyl oder

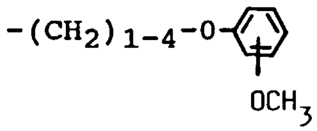

N-substituiert sein kann.

**4.** Verbindung I nach, Anspruch 1 oder 2, worin A Indolo ist und die anderen Substituenten wie in Anspruch 1 definiert sind, vorzugsweise $NR^3R^4$ entweder Morpholinyl ist oder in $NR^3R^4$
$R^3$ Wasserstoff ist und $R^4$ Alkyl mit 1 bis 4 Kohlenstoffatomen, das wie in Anspruch 1 definiert substituiert sein kann.

**5.** Verbindung I nach Anspruch 1, 2 oder 3, worin A Benzo oder Thieno ist, und, wenn A Benzo ist m 2 ist und die beiden $R^2$ unabhängig voneinander Methoxy, Hydroxy, Benzyloxy, Methyl oder Chlor oder zusammen $-OCH_20$-sind, wobei die beiden $R^2$ in den Positionen 6 und 7 stehen.

**6.** Verbindung I nach Anspruch 5, worin $R^2$ Methoxy, Hydroxy, Benzyloxy oder Methyl ist.

**7.** Verbindung I nach Anspruch 5 oder 6, worin die durch $R^2$ substituierte Gruppe A Thieno, 6,7-Dihydroxybenzo oder vorzugsweise 6,7-Dimethoxybenzo ist.

**8.** Verbindung I nach einen der Ansprüche 1-7, worin $R^1$ 3-Thienyl ist.

**9.** Verbindung I nach einem der Ansprüche 1 bis 7, worin $R^1$ tert. Butyl ist.

**10.** Verbindung I nach einem der Ansprüche 1 bis 9, worin $NR^3R^4$ eine der folgenden Bedeutungen hat

a) in $NR^3R^4$ ist $R^3$ Wasserstoff und $R^4$ $C_1-C_6$ Alkyl;
b) in $NR^3R^4$ ist $R^3$ Wasserstoff und $R^4$ verzweigtes oder unverzweigtes Alkinyl mit 3 bis 6 Kohlenstoffatomen
c) in $NR^3R^4$ ist $R^3$ Wasserstoff und $R^4$ verzweigtes oder unverzweigtes Alkyl mit 1-4 Kohlenstoffatomen, wobei das Alkyl substituiert ist durch

Methoxy,
Dimethylamino,
Pyrrolidinyl, N-Methylpyrrolidinyl,

Morpholino,
Thienyl,
Adamantyl,
Pyridyl,
N-Benzylpiperidyl,
Cyclohexyl,
Phenoxy,
Naphthyloxy oder 1 oder 2 Phenyl, wobei dieses Phenyl (wenn nur eine Phenylgruppe vorliegt) oder das in der Phenoxygruppe enthaltene Phenyl ein-, zwei- oder dreifach durch Methoxy, Ethoxy, Benzyloxy, Halogen, $CF_3$, $N_3$, Methyl, tert. Butyl, $-SO_2NH_2$, oder durch die Brücke $-O-CH_2-O-$ substituiert sein kann; oder $R^3$ Wasserstoff bedeutet und $R^4$ Cyclohexyl, Phenyl, Fluorophenyl, Pyridyl oder N-Benzylpiperidyl;

d) in $NR^3R^4$ sind $R^3$ und $R^4$ unabhängig voneinander Methyl, Ethyl, $(CH_2)_{1-4}$ Phenyl (worin die Phenylgruppe substituiert sein kann wie die in (c) angegebene Phenylgruppe) oder

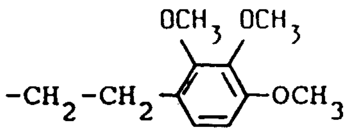

;

e) $R^3$ und $R^4$ gemeinsam mit dem Stickstoffatom, an das sie gebunden sind, sind

Piperidinyl,
Morpholinyl, Thiomorpholinyl,
oder
Piperazinyl, wobei der Piperazinylring gegebenenfalls durch Methyl oder Benzyl N-substituiert sein kann.

**11.** Verbindung I nach Anspruch 10, worin $NR^3R^4$ eine der folgenden Bedeutungen hat

a) in $NR^3R^4$ ist $R^3$ Wasserstoff und $R^4$ $C_2$-$C_6$ Alkyl;
b) in $NR^3R^4$ ist $R^3$ Wasserstoff und $R^4$ ist $CH_2CCH$;
c) in $NR^3R^4$ ist $R^3$ Wasserstoff und $R^4$ verzweigtes oder unverzweigtes Alkyl mit 2-4 Kohlenstoffatomen, wobei das Alkyl substituiert ist durch

Methoxy,
Dimethylamino,
N-Methylpyrrolidinyl,
Thienyl,
Adamantyl,
Phenoxy,
Naphthyloxy oder 1 oder 2 Phenyl, wobei dieses Phenyl (wenn nur eine Phenylgruppe vorliegt) oder das in der Phenoxygruppe enthaltene Phenyl ein-, zwei- oder dreifach durch Methoxy, Ethoxy, $N_3$, Methyl, tert. Butyl oder $-SO_2NH_2$ substituiert sein kann;

d) in $NR^3R^4$ sind $R^3$ und $R^4$ unabhängig voneinander Methyl, Ethyl, $(CH_2)_{1-4}$ Phenyl (worin die Phenylgruppe durch F substituiert sein kann) oder

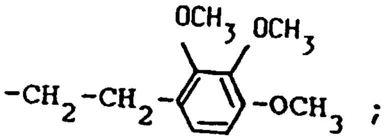

e) $R^3$ und $R^4$ gemeinsam mit dem Stickstoffatom, an das sie gebunden sind, sind

Piperazinyl, N-subsitutiert durch Methyl oder Benzyl.

**12.** Verbindung I nach Anspruch 10, worin $NR^3R^4$ eine der folgenden Bedeutungen hat.

a) in $NR^3R^4$ ist $R^3$ Wasserstoff und $R^4$ ist Ethyl, tert. Butyl oder $(CH_2)_{1\ oder\ 2}$-$C(CH_3)_3$;

b) $NR^3R^4$ ist $NHCH_2CCH$;

c) in $NR^3R^4$ ist $R^3$ Wasserstoff und $R^4$ ist Ethyl, Propyl oder Methylpropyl, das durch Phenyl substituiert ist, das ein-, zwei- oder dreifach durch Methyl oder Methoxy oder einfach durch tert. Butyl substituiert ist;

d) in $NR^3R^4$ sind $R^3$ und $R^4$ gleich, nämlich

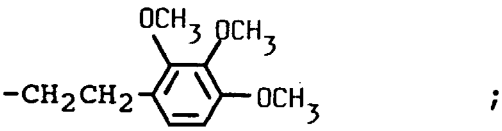

e) $NR^3R^4$ ist

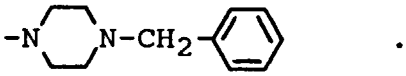

**13.** Verbindung I nach Anspruch 10, worin $R^3$ Wasserstoff oder $(C_1$-$C_4)$Alkyl-phenyl ist und $R^4$ $(C_1$-$C_4)$ Alkyl-phenyl, wobei in diesen Gruppen jeweils Phenyl monosubstituiert ist durch Halogen, $CF_3$, Methoxy oder Ethoxy.

**14.** Verbindung I nach einen der Ansprüche 1 bis 9, worin $R^3$ Wasserstoff ist und $R^4$

a) $(C_1$-$C_3)$Alkyl ist, das durch Phenyl substituiert ist, das durch $CF_3$, Cl, F, tert. Butyl oder $CH_3$ substituiert sein kann,
oder
b) 2,2-Diphenylethyl oder 3,3-Diphenylpropyl ist, oder
c) Cyclohexyl ist.

**15.** Verbindung nach Anspruch 9 der Formel

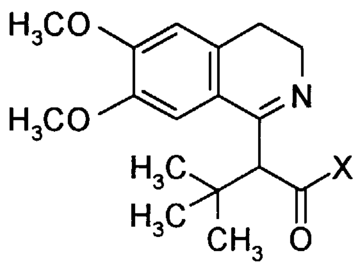

worin X die Gruppe

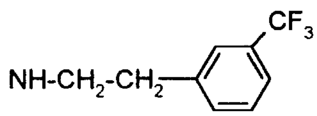

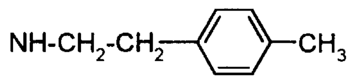

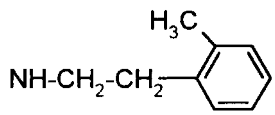

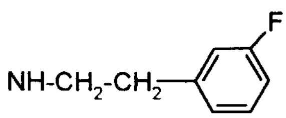

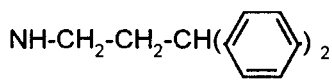

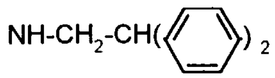

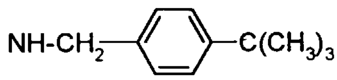

oder

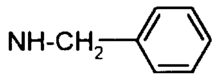

ist.

16. Verfahren zur Herstellung einer Verbindung nach einem der Ansprüche 1 bis 15, dadurch gekennzeichnet, daß man ein Malonsäurediamid der allgemeinen Formel IV

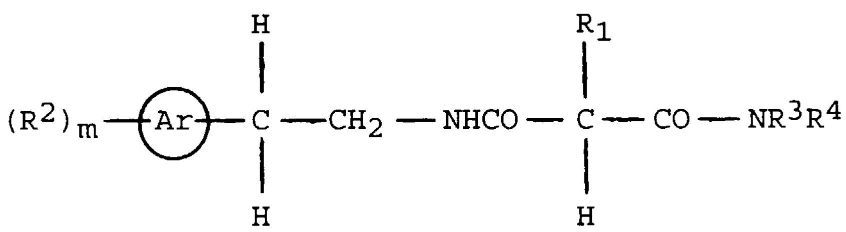

in der $R^1$, $R^2$, $R^3$, $R^4$ und m, wie in einem der Ansprüche 1 bis 15 definiert sind und Ar Phenyl, Indolyl oder 2- oder 3-Thienyl bedeutet, in Gegenwart eines Kondensationsmittels cyclisiert und gewünschtenfalls in ihr Salz überführt.

17. Pharmazeutische Zubereitung enthaltend eine Verbindung gemäß einem der Ansprüche 1 bis 15.

18. Verfahren zur Herstellung pharmazeutischer Präparate nach Anspruch 17, dadurch gekennzeichnet, daß man eine Verbindung gemäß einem der Ansprüche 1 bis 15 mit üblichen galenischen Hilfs- und/oder Trägerstoffen zu üblichen pharmazeutischen Anwendungsformen verarbeitet.

19. Verwendung einer Verbindung der Formel I, wie sie in einem der Ansprüche 1 bis 15 definiert ist, für die Herstellung eines cardioprotektiven Mittels, eines hirnprotektiven Mittels oder eines Mittels für die Behandlung chronisch inflammatorischer Prozesse, eines Mittels mit antiproliferativer Wirkung oder eines Mittels zur Behandlung von Colitis Ulcerosa oder Morbus Crohn.

**Claims**

1. Compound of general formula I

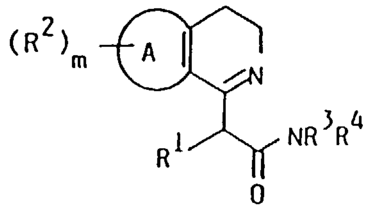

I

wherein

A denotes a benzo, indolo or thieno group;
wherein, if A is benzo, m is 2 or 3, and the substituents $R^2$ independently of each other denote hydroxy, $(C_{1-4})$alkoxy, benzyloxy, halogen (F, Cl, Br, I), $(C_{1-4})$alkyl, methanesulphonyloxy or methanesulphonamido, or two adjacent substituents $R^2$ may together represent $-O-CH_2-O-$ or $-O-CH_2-CH_2-O-$; and if A is indolo or thieno, m is zero;

$R^1$ denotes thienyl or the group

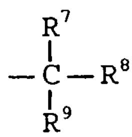

wherein

$R^7$, $R^8$ and $R^9$ independently of one another may represent methyl, ethyl, propyl, phenyl or benzyl, whilst not more than 2 of the substituents can simultaneously represent phenyl or benzyl;

$R^3$ and $R^4$ independently of each other have one of the following meanings:

(a) hydrogen,
(b) branched or unbranched $C_{3-6}$-alkenyl,
(c) branched or unbranched $C_{3-6}$-alkynyl, or
(d) branched or unbranched $C_{1-12}$-alkyl, wherein the alkyl may be substituted by

hydroxy,
$(C_{1-4})$alkoxy,
di$(C_{1-4})$alkylamino,
furyl,
pyridyl,
pyrrolidinyl, N-methylpyrrolidinyl,
morpholino,
indolyl,
nitrilo,
thienyl,
adamantyl,
cyclohexyl,
phenoxy,
naphthyloxy or phenyl, [whilst this phenyl or the phenyl contained in the phenoxy group may be mono-, di- or trisubstituted by hydroxy, $(C_{1-4})$alkoxy, benzyloxy, halogen (F, Cl, Br, I), $CF_3$, $N_3$, CN, $(C_{1-4})$alkyl, adamantyl, $-SO_2NH_2$, $-NHCOCH_3$, $-NHSO_2CH_3$ or $CH_3SO_2O-$ or by the bridge $-O-CH_2-O-$]; or by two unsubstituted phenyl groups;
or $R^3$ represents hydrogen and $R^4$ represents cyclohexyl, phenyl (whilst this phenyl may be mono-, di-

or trisubstituted by hydroxy, $(C_{1-4})$alkoxy, benzyloxy, halogen (F, Cl, Br, I), $CF_3$, $N_3$, $(C_{1-4})$alkyl, adamantyl, $-SO_2NH_2$, $-NHCOCH_3$, $-NHSO_2CH_3$ or $CH_3SO_2O-$ or by the bridge $-O-CH_2-O-$); pyridyl or N-benzylpiperidyl;
or $R^3$ and $R^4$ together with the nitrogen atom to which they are bound represent pyrrolidinyl, piperidinyl, morpholinyl, thiomorpholinyl or piperazinyl, whilst the piperazinyl ring may optionally be N-substituted by methyl, unsubstituted phenyl, mono- or di$(C_{1-4})$alkoxyphenyl, cyano-substituted phenyl, pyrimidinyl, phenyl$(C_{1-4})$alkyl, $(C_{1-4})$ alkylphenyl or

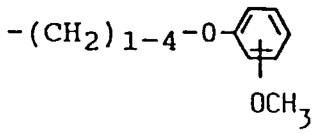

;

or the salts thereof with physiologically acceptable acids or complex-forming agents.

**2.** Compounds according to claim 1 wherein

$R^3$ and $R^4$ independently of each other represent

(a) hydrogen,
(b) branched or unbranched $C_{3-6}$-alkenyl,
(c) branched or unbranched $C_{3-6}$-alkynyl, or
(d) branched or unbranched $C_{1-12}$-alkyl, wherein the alkyl may be substituted by

hydroxy,
$(C_{1-4})$alkoxy,
di$(C_{1-4})$alkylamino,
furyl,
pyridyl,
pyrrolidinyl, N-methylpyrrolidinyl,
morpholino,
indolyl,
nitrilo,
thienyl,
adamantyl,
cyclohexyl,
phenoxy,
naphthyloxy or phenyl [whilst this phenyl or the phenyl contained in the phenoxy group may be mono-, di- or trisubstituted by hydroxy, $(C_{1-4})$alkoxy, benzyloxy, halogen (F, Cl, Br, I), $CF_3$, $N_3$, $(C_{1-4})$alkyl, adamantyl, $-SO_2NH_2$, $-NHCOCH_3$, $-NHSO_2CH_3$ or $CH_3SO_2O-$ or by the bridge $-O-CH_2-O-$]
or $R^3$ denotes hydrogen and $R^4$ denotes cyclohexyl, phenyl [whilst this phenyl may be mono-, di- or trisubstituted by hydroxy, $(C_{1-4})$alkoxy, benzyloxy, halogen (F, Cl, Br, I), $CF_3$, $N_3$, $(C_{1-4})$alkyl, adamantyl, $-SO_2NH_2$, $-NHCOCH_3$, $-NHSO_2CH_3$ or $CH_3SO_2O-$ or by the bridge $-O-CH_2-O-$], pyridyl or N-benzylpiperidyl;
or $R^3$ and $R^4$ together with the nitrogen atom to which they are bound represent pyrrolidinyl, piperidinyl, morpholinyl, thiomorpholinyl or piperazinyl, whilst the piperazinyl ring may optionally be N-substituted by methyl, unsubstituted phenyl, mono- or di$(C_{1-4})$alkoxyphenyl, pyrimidinyl, phenyl$(C_{1-4})$alkyl or

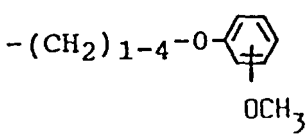

;

or the salts thereof with physiologically acceptable acids or complex-forming agents.

**3.** Compounds of general formula I according to claim 1 or 2 wherein

A denotes a benzo or thieno group;
wherein, if A is benzo, m is 2, the two $R^2$s are in positions 6 and 7 and independently of each other denote hydroxy, $(C_{1-4})$alkoxy, benzyloxy, halogen (F, Cl, Br, I), $(C_{1-4})$alkyl, methanesulphonyloxy or methanesulphonamido, or two adjacent substituents $R^2$ may together represent $-O-CH_2-O-$ or $-O-CH_2-CH_2-O-$; and if A is thieno, m is zero;

$R^1$ is as defined in claim 1;

$R^3$ and $R^4$ independently of each other denote

(a) hydrogen,
(b) branched or unbranched $C_{3-6}$-alkenyl,
(c) branched or unbranched $C_{3-6}$-alkynyl, or
(d) branched or unbranched $C_{1-12}$-alkyl, wherein the alkyl may be substituted by

hydroxy,
$(C_{1-4})$alkoxy,
di$(C_{1-4})$alkylamino,
furyl,
pyridyl,
pyrrolidinyl, N-methylpyrrolidinyl,
morpholino,
indolyl,
nitrilo,
thienyl,
adamantyl,
cyclohexyl,
phenoxy,
naphthyloxy or phenyl, whilst this phenyl or the phenyl contained in the phenoxy group may be mono-, di- or trisubstituted by hydroxy, $(C_{1-4})$alkoxy, benzyloxy, halogen (F, Cl, Br, I), $CF_3$, $N_3$, $(C_{1-4})$alkyl, adamantyl, $-SO_2NH_2$ or $-NHCOCH_3$, or by the bridge $-O-CH_2-O-$;
or $R^3$ represents hydrogen and $R^4$ represents cyclohexyl, phenyl, fluorophenyl, pyridyl or N-benzylpiperidyl;
or $R^3$ and $R^4$ together with the nitrogen atom to which they are bound represent pyrrolidinyl, piperidinyl, morpholinyl, thiomorpholinyl or piperazinyl, whilst the piperazinyl ring may optionally be N-substituted by methyl, unsubstituted phenyl, mono- or di$(C_{1-4})$alkoxyphenyl, pyrimidinyl, phenyl$(C_{1-4})$alkyl or

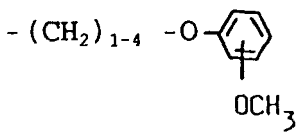

.

**4.** Compound I according to claim 1 or 2, wherein A is indolo and the other substituents are defined as in claim 1, preferably $NR^3R^4$ is either morpholinyl or in $NR^3R^4$, $R^3$ is hydrogen and $R^4$ is $C_{1-4}$-alkyl which may be substituted as defined in claim 1.

**5.** Compound I according to claim 1, 2 or 3, wherein A is benzo or thieno and, if A is benzo, m is 2 and the two $R^2$s independently of each other represent methoxy, hydroxy, benzyloxy, methyl or chlorine or together represent $-OCH_2O-$, the two $R^2$s being in positions 6 and 7.

**6.** Compound I according to claim 5, wherein $R^2$ is methoxy, hydroxy, benzyloxy or methyl.

**7.** Compound I according to claim 5 or 6, wherein the group A substituted by $R^2$ is thieno, 6,7-dihydroxybenzo or preferably 6,7-dimethoxybenzo.

**8.** Compound I according to one of claims 1 to 7, wherein $R^1$ is 3-thienyl.

**9.** Compound I according to one of claims 1 to 7, wherein $R^1$ is tert.butyl.

**10.** Compound I according to one of claims 1 to 9, wherein $NR^3R^4$ has one of the following meanings:

a) in $NR^3R^4$, $R^3$ is hydrogen and $R^4$ is $C_{1-6}$-alkyl;
b) in $NR^3R^4$, $R^3$ is hydrogen and $R^4$ is branched or unbranched alkynyl having 3 to 6 carbon atoms
c) in $NR^3R^4$, $R^3$ is hydrogen and $R^4$ is branched or unbranched alkyl having 1 to 4 carbon atoms, the alkyl being substituted by

methoxy,
dimethylamino,
pyrrolidinyl, N-methypyrrolidinyl,
morpholino,
thienyl,
adamantyl,
pyridyl,
N-benzylpiperidyl,
cyclohexyl,
phenoxy,
naphthyloxy or 1 or 2 phenyl, whilst this phenyl (if only one phenyl group is present) or the phenyl contained in the phenoxy group may be mono-, di- or trisubstituted by methoxy, ethoxy, benzyloxy, halogen, $CF_3$, $N_3$, methyl, tert.butyl, $-SO_2NH_2$, or by the bridge $-O-CH_2-O-$;
or $R^3$ denotes hydrogen and $R^4$ denotes cyclohexyl, phenyl, fluorophenyl, pyridyl or N-benzylpiperidyl;

d) in $NR^3R^4$, $R^3$ and $R^4$ independently of each other represent methyl, ethyl, $(CH_2)_{1-4}$-phenyl (wherein the phenyl group may be substituted like the phenyl group specified in (c))
or

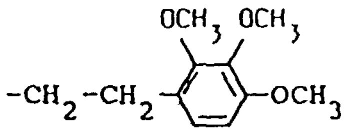
;

e) $R^3$ and $R^4$ together with the nitrogen atom to which they are bound denote piperidinyl, morpholinyl, thiomorpholinyl or piperazinyl, whilst the piperazinyl ring may optionally be N-substituted by methyl or benzyl.

**11.** Compound I according to claim 10, wherein $NR^3R^4$ has one of the following meanings:

a) in $NR^3R^4$, $R^3$ is hydrogen and $R^4$ is $C_{2-6}$-alkyl;
b) in $NR^3R^4$, $R^3$ is hydrogen and $R^4$ is $CH_2CCH$;
c) in $NR^3R^4$, $R^3$ as hydrogen and $R^4$ is branched or unbranched $C_{2-4}$-alkyl, wherein the alkyl is substituted by

methoxy,
dimethylamino,
N-methypyrrolidinyl,
thienyl,
adamantyl,
phenoxy,
naphthyloxy or 1 or 2 phenyl, whilst this phenyl (if there is only one phenyl group present) or the phenyl contained in the phenoxy group may be mono-, di- or trisubstituted by methoxy, ethoxy, $N_3$, methyl,

tert.butyl or $-SO_2NH_2$;

d) in $NR^3R^4$, $R^3$ and $R^4$ independently of each other represent methyl, ethyl, $(CH_2)_{1-4}$-phenyl (wherein the phenyl group may be substituted by F) or

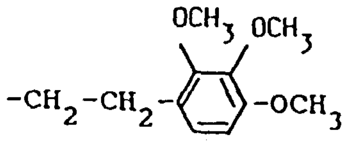 ;

e) $R^3$ and $R^4$ together with the nitrogen atom to which they are bound are piperazinyl, N-substituted by methyl or benzyl.

**12.** Compound I according to claim 10, wherein $NR^3R^4$ has one of the following meanings:

a) in $NR^3R^4$, $R^3$ is hydrogen and $R^4$ is ethyl, tert.butyl or $(CH_2)_{1\ or\ 2}$-$C(CH_3)_3$;

b) $NR^3R^4$ is $NHCH_2CCH$;

c) in $NR^3R^4$, $R^3$ is hydrogen and $R^4$ is ethyl, propyl or methylpropyl which is substituted by phenyl, which may be mono-, di- or trisubstituted by methyl or methoxy or monosubstituted by tert.butyl;

d) in $NR^3R^4$, $R^3$ and $R^4$ are identical, namely

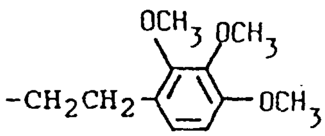 ;

e) $NR^3R^4$ is

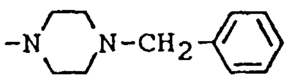 .

**13.** Compound I according to claim 10, wherein $R^3$ is hydrogen or $(C_{1-4})$alkyl-phenyl and $R^4$ is $(C_{1-4})$alkylphenyl, whilst in these groups phenyl is mono-substituted by halogen, $CF_3$, methoxy or ethoxy.

**14.** Compound I according to one of claims 1 to 9, wherein $R^3$ is hydrogen and $R^4$ is

a) $(C_{1-3})$alkyl which is substituted by phenyl, which may be substituted by $CF_3$, Cl, F, tert.butyl or $CH_3$; or

b) 2,2-diphenylethyl or 3,3-diphenylpropyl; or

c) cyclohexyl.

**15.** Compound according to claim 9 of the formula

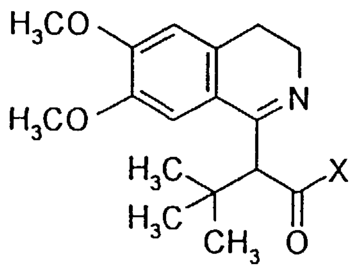

wherein X is the group

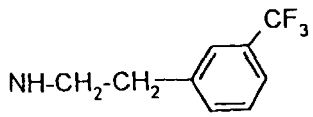

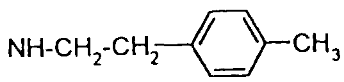

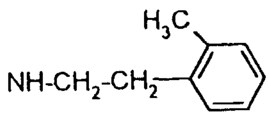

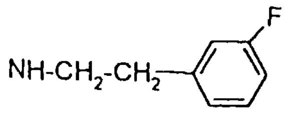

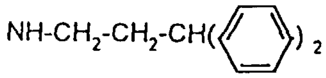

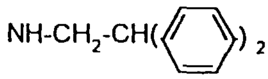

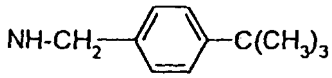

or

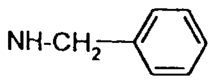

**16.** Process for preparing a compound according to one of claims 1 to 15, characterised in that a malonic acid diamide of general formula IV

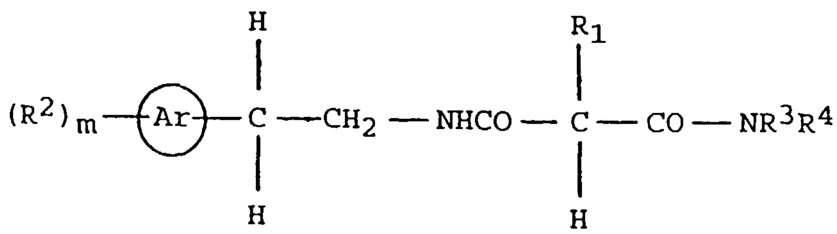

wherein $R^1$, $R^2$, $R^3$, $R^4$ and m are defined as in one of claims 1 to 15 and Ar represents phenyl, indolyl or 2- or 3-thienyl, is cyclised in the presence of a condensing agent and, if desired, converted into a salt thereof.

**17.** Pharmaceutical preparation containing a compound according to one of claims 1 to 15.

**18.** Process for preparing pharmaceutical preparations according to claim 17, characterised in that compounds according to one of claims 1 to 15 are processed with conventional galenic excipients and/or carriers to produce conventional pharmaceutical formulations.

**19.** Use of a compound of formula I as defined in one of claims 1 to 15 for preparing a cardioprotective agent, a cerebroprotective agent or an agent for treating chronically inflammatory processes, an agent with an antiproliferative effect or an agent for treating ulcerative colitis or Crohn's disease.

## Revendications

**1.** Composé de formule générale I

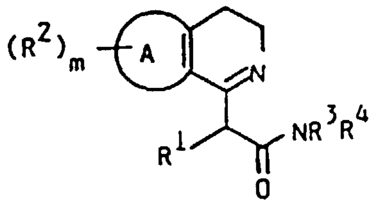

I

où

A représente un reste benzo, indolo ou thiéno;
où, quand A est benzo, m est 2 ou 3,
et les substituants $R^2$ peuvent être indépendamment les uns des autres hydroxyle, alcoxy en $C_1$-$C_4$, benzyloxy, halogène (F, Cl, Br, I), alkyle en $C_1$-$C_4$, méthanesulfonyloxy ou méthanesulfonamido, ou deux substituants $R^2$ voisins peuvent être ensemble -O-$CH_2$-O- ou -O-$CH_2$-$CH_2$-O-;
et quand A est indolo ou thiéno, m est zéro;
$R^1$ représente thiényle ou le groupe

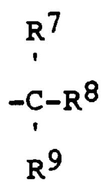

où
$R^7$, $R^8$ et $R^9$ peuvent être indépendamment les uns des autres méthyle, éthyle, propyle, phényle ou benzyle, où au plus 2 des substituants peuvent être en même temps phényle ou benzyle;
$R^3$ et $R^4$ représentent indépendamment l'un de l'autre

(a) l'hydrogène,
(b) alcényle ramifié ou non ramifié ayant 3 à 6 atomes de carbone,
(c) alcynyle ramifié ou non ramifié ayant 3 à 6 atomes de carbone, ou
(d) alkyle ramifié ou non ramifié ayant 1-12 atomes de carbone, où l'alkyle peut être substitué par

hydroxyle,
alcoxy en $C_1$-$C_4$,
dialkyle en $C_1$-$C_4$-amino,
furyle,
pyridyle,
pyrrolidinyle, N-méthylpyrrolidinyle,
morpholino,
indolyle,
nitrilo,
thiényle,
adamantyle,
cyclohexyle,
phénoxy,
naphtyloxy ou phényle [où ce phényle ou le phényle contenu dans le groupe phénoxy peut être substitué une, deux ou trois fois par hydroxyle, alcoxy en $C_1$-$C_4$, benzyloxy, halogène (F, Cl, Br, I), $CF_3$, $N_3$, CN, alkyle en $C_1$-$C_4$, adamantyle, -$SO_2NH_2$, -$NHCOCH_3$, -$NHSO_2CH_3$ ou $CH_3SO_2O$- ou par le pont -O-$CH_2$-O-] ou par deux groupes phényle non substitués;
ou $R^3$ représente l'hydrogène et $R^4$ représente cyclohexyle, phényle [où ce phényle peut être substitué une, deux ou trois fois par hydroxyle, alcoxy en $C_1$-$C_4$, benzyloxy, halogène (F, Cl, Br, I), $CF_3$, $N_3$,

alkyle en $C_1$-$C_4$, adamantyle, $-SO_2NH_2$, $-NHCOCH_3$, $-NHSO_2CH_3$ ou $CH_3SO_2O$- ou par le pont -O-$CH_2$-O-], pyridyle ou N-benzylpipéridyle;

ou $R^3$ et $R^4$ représentent avec l'atome d'azote auquel ils sont liés

pyrrolidinyle,

pipéridinyle,

morpholinyle, thiomorpholinyle,

ou

pipérazinyle, où le cycle pipérazinyle peut éventuellement être N-substitué par méthyle, phényle non substitué, mono- ou dialcoxy en $C_1$-$C_4$-phényle, phényle cyano-substitué, pyrimidinyle, phényl-alkyle en $C_1$-$C_4$, alkyle en $C_1$-$C_4$-phényle ou

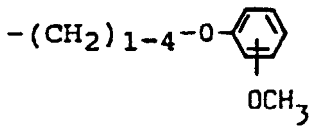

ou ses sels avec des acides ou complexants physiologiquement acceptables.

**2.** Composé selon la revendication 1 où

$R^3$ et $R^4$ représentent indépendamment l'un de l'autre

(a) l'hydrogène,
(b) alcényle ramifié ou non ramifié ayant 3 à 6 atomes de carbone,
(c) alcynyle ramifié ou non ramifié ayant 3 à 6 atomes de carbone, ou
(d) alkyle ramifié ou non ramifié ayant 1-12 atomes de carbone, où l'alkyle peut être substitué par

hydroxyle,

alcoxy en $C_1$-$C_4$,

dialkyle en $C_1$-$C_4$-amino,

furyle,

pyridyle,

pyrrolidinyle, N-méthylpyrrolidinyle,

morpholino,

indolyle,

nitrilo,

thiényle,

adamantyle,

cyclohexyle,

phénoxy,

naphtyloxy ou phényle [où ce phényle ou le phényle contenu dans le groupe phénoxy peut être substitué une, deux ou trois fois par hydroxyle, alcoxy en $C_1$-$C_4$, benzyloxy, halogène (F, Cl, Br, I), $CF_3$, $N_3$, alkyle en $C_1$-$C_4$, adamantyle, $-SO_2NH_2$, $-NHCOCH_3$, $-NHSO_2CH_3$ ou $CH_3SO_2O$- ou par le pont -O-$CH_2$-O-];

ou $R^3$ représente l'hydrogène et $R^4$ représente cyclohexyle, phényle [où ce phényle peut être substitué une, deux ou trois fois par hydroxyle, alcoxy en $C_1$-$C_4$, benzyloxy, halogène (F, Cl, Br, I), $CF_3$, $N_3$, alkyle en $C_1$-$C_4$, adamantyle, $-SO_2NH_2$, $-NHCOCH_3$, $-NHSO_2CH_3$ ou $CH_3SO_2O$- ou par le pont -O-$CH_2$-O-], pyridyle ou N-benzylpipéridyle;

ou $R^3$ et $R^4$ représentent avec l'atome d'azote auquel ils sont liés

pyrrolidinyle,

pipéridinyle,

morpholinyle, thiomorpholinyle,

ou

pipérazinyle, où le cycle pipérazinyle peut éventuellement être N-substitué par méthyle, phényle non

substitué, mono- ou dialcoxy en $C_1$-$C_4$-phényle, pyrimidinyle, phényl-alkyle en $C_1$-$C_4$, ou

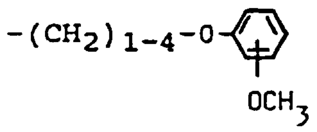

ou ses sels avec des acides ou complexants physiologiquement acceptables.

**3.** Composé de formule générale I selon la revendication 1 ou 2
où

A représente un reste benzo ou thiéno;
où, quand A est benzo, m est 2, les deux $R^2$ sont dans les positions 6 et 7 et peuvent être indépendamment l'un de l'autre hydroxyle, alcoxy en $C_1$-$C_4$, benzyloxy, halogène (F, Cl, Br, I), alkyle en $C_1$-$C_4$, méthanesulfonyloxy ou méthanesulfonamido, ou deux substituants $R^2$ voisins peuvent être ensemble -O-$CH_2$-O- ou -O-$CH_2$-$CH_2$-O-;
et quand A est thiéno, m est zéro;
$R^1$ est défini comme dans la revendication 1,
$R^3$ et $R^4$ représentent indépendamment l'un de l'autre

(a) l'hydrogène,
(b) alcényle ramifié ou non ramifié ayant 3 à 6 atomes de carbone,
(c) alcynyle ramifié ou non ramifié ayant 3 à 6 atomes de carbone, ou
(d) alkyle ramifié ou non ramifié ayant 1-12 atomes de carbone, où l'alkyle peut être substitué par

hydroxyle,
alcoxy en $C_1$-$C_4$,
dialkyle en $C_1$-$C_4$-amino,
furyle,
pyridyle,
pyrrolidinyle, N-méthylpyrrolidinyle,
morpholino,
indolyle,
nitrilo,
thiényle,
adamantyle,
cyclohexyle,
phénoxy,
naphtyloxy ou phényle, où ce phényle ou le phényle contenu dans le groupe phénoxy peut être substitué une, deux ou trois fois par hydroxyle, alcoxy en $C_1$-$C_4$, benzyloxy, halogène (F, Cl, Br, I), $CF_3$, $N_3$, alkyle en $C_1$-$C_4$, adamantyle, -$SO_2NH_2$ ou -$NHCOCH_3$, ou par le pont -O-$CH_2$-O-];
ou $R^3$ représente l'hydrogène et $R^4$ représente cyclohexyle, phényle, fluorophényle, pyridyle ou N-benzylpipéridyle;
ou $R^3$ et $R^4$ représentent avec l'atome d'azote auquel ils sont liés
pyrrolidinyle,
pipéridinyle,
morpholinyle, thiomorpholinyle,
ou
pipérazinyle, où le cycle pipérazinyle peut éventuellement être N-substitué par méthyle, phényle non substitué, mono- ou dialcoxy en $C_1$-$C_4$-phényle, pyrimidinyle, phényl-alkyle en $C_1$-$C_4$, ou

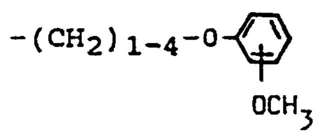

4. Composé I selon la revendication 1 ou 2 où A est indolo et les autres substituants sont définis comme dans la revendication 1, de préférence $NR^3R^4$ est morpholinyle ou dans $NR^3R^4$ $R^3$ est l'hydrogène et $R^4$ est alkyle ayant 1 à 4 atomes de carbone, qui peut être substitué comme défini dans la revendication 1.

5. Composé I selon la revendication 1, 2 ou 3 où A est benzo ou thiéno et, quand A est benzo, m est 2 et les deux $R^2$ sont indépendamment l'un de l'autre méthoxy, hydroxyle, benzyloxy, méthyle ou chloro ou sont ensemble $-OCH_2O-$, où les deux $R^2$ sont dans les positions 6 et 7.

6. Composé I selon la revendication 5 où $R^2$ est méthoxy, hydroxyle, benzyloxy ou méthyle.

7. Composé I selon la revendication 5 ou 6 où le groupe A substitué par $R^2$ est thiéno, 6,7-dihydroxybenzo ou de préférence 6,7-diméthoxybenzo.

8. Composé I selon l'une des revendications 1-7 où $R^1$ est 3-thiényle.

9. Composé I selon l'une des revendications 1 à 7 où $R^1$ est tert.butyle.

10. Composé I selon l'une des revendications 1 à 9 où $NR^3R^4$ a l'une des significations suivantes

a) dans $NR^3R^4$ $R^3$ est l'hydrogène et $R^4$ est alkyle en $C_1$-$C_6$;
b) dans $NR^3R^4$ $R^3$ est l'hydrogène et $R^4$ est alcynyle ramifié ou non ramifié ayant 3 à 6 atomes de carbone
c) dans $NR^3R^4$ $R^3$ est l'hydrogène et $R^4$ est alkyle ramifié ou non ramifié ayant 1-4 atomes de carbone, où l'alkyle est substitué par

méthoxy,
diméthylamino,
pyrrolidinyle, N-méthylpyrrolidinyle,
morpholino,
thiényle,
adamantyle,
pyridyle,
N-benzylpipéridyle,
cyclohexyle,
phénoxy,
naphtyloxy ou 1 ou 2 phényles, où ce phényle (quand il n'y a qu'un seul groupe phényle) ou le phényle contenu dans le groupe phénoxy peut être substitué une, deux ou trois fois par méthoxy, éthoxy, benzyloxy, halogène, $CF_3$, $N_3$, méthyle, tert.butyle, $-SO_2NH_2$, ou par le pont $-O-CH_2-O-$;
ou $R^3$ représente l'hydrogène et $R^4$ représente cyclohexyle, phényle, fluorophényle, pyridyle ou N-benzylpipéridyle;

d) dans $NR^3R^4$ $R^3$ et $R^4$ sont indépendamment l'un de l'autre méthyle, éthyle, $(CH_2)_{1-4}$ phényle (où le groupe phényle peut être substitué comme le groupe phényle indiqué en (c))
ou

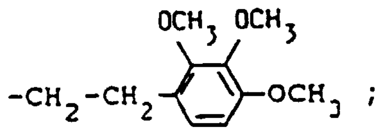

e) $R^3$ et $R^4$ sont, avec l'atome d'azote auquel ils sont liés,

pipéridinyle,
morpholinyle, thiomorpholinyle,
ou
pipérazinyle, où le cycle pipérazinyle peut éventuellement être N-substitué par méthyle ou benzyle.

**11.** Composé I selon la revendication 10 où $NR^3R^4$ a l'une des significations suivantes

a) dans $NR^3R^4$ $R^3$ est l'hydrogène et $R^4$ est alkyle en $C_2$-$C_6$;
b) dans $NR^3R^4$ $R^3$ est l'hydrogène et $R^4$ est $CH_2CCH$;
c) dans $NR^3R^4$ $R^3$ est l'hydrogène et $R^4$ est alkyle ramifié ou non ramifié ayant 2-4 atomes de carbone, où l'alkyle est substitué par

méthoxy,
diméthylamino,
N-méthylpyrrolidinyle,
thiényle,
adamantyle,
phénoxy,
naphtyloxy ou 1 ou 2 phényles, où ce phényle (quand il n'y a qu'un seul groupe phényle) ou le phényle contenu dans le groupe phénoxy peut être substitué une, deux ou trois fois par méthoxy, éthoxy, $N_3$, méthyle, tert.butyle ou $-SO_2NH_2$;

d) dans $NR^3R^4$ $R^3$ et $R^4$ sont indépendamment l'un de l'autre méthyle, éthyle, $(CH_2)_{1-4}$ phényle (où le groupe phényle peut être substitué par F) ou

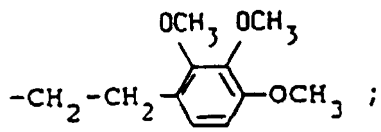

e) $R^3$ et $R^4$ sont, avec l'atome d'azote auquel ils sont liés,

pipérazinyle, N-substitué par méthyle ou benzyle.

**12.** Composé I selon la revendication 10 où $NR^3R^4$ a l'une des significations suivantes:

a) dans $NR^3R^4$ $R^3$ est l'hydrogène et $R^4$ est éthyle, tert.butyle ou $(CH_2)_{1\text{ ou }2}$-$C(CH_3)_3$;
b) $NR^3R^4$ est $NHCH_2CCH$;
c) dans $NR^3R^4$ $R^3$ est l'hydrogène et $R^4$ est éthyle, propyle ou méthylpropyle qui est substitué par phényle qui est substitué une, deux ou trois fois par méthyle ou méthoxy ou une fois par tert.butyle;
d) dans $NR^3R^4$ $R^3$ et $R^4$ sont identiques, à savoir

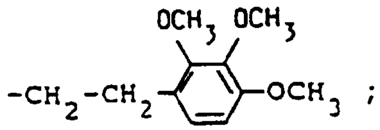

e) $NR^3R^4$ est

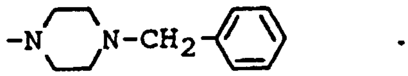

13. Composé I selon la revendication 10 où $R^3$ est l'hydrogène ou alkyle en $C_1$-$C_4$-phényle et $R^4$ est alkyle en $C_1$-$C_4$-phényle, où dans chacun de ces groupes phényle est monosubstitué par halogène, $CF_3$, méthoxy ou éthoxy.

14. Composé I selon l'une des revendications 1 à 9 où $R^3$ est l'hydrogène et $R^4$ est

a) alkyle en $C_1$-$C_3$ qui est substitué par phényle qui peut être substitué par $CF_3$, Cl, F, tert.butyle ou $CH_3$, ou
b) 2,2-diphényléthyle ou 3,3-diphénylpropyle, ou
c) cyclohexyle.

15. Composé selon la revendication 9 de formule

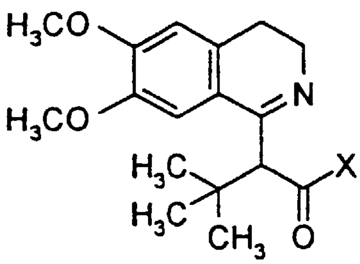

où X est le groupe

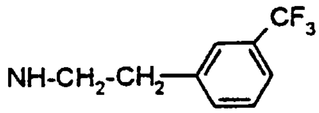

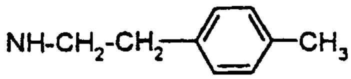

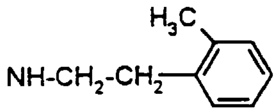

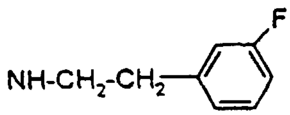

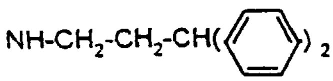

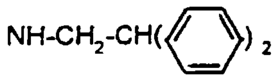

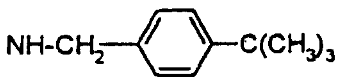

ou

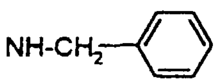

**16.** Procédé de préparation d'un composé selon l'une des revendications 1 à 15 caractérisé en ce que l'on cyclise un diamide d'acide malonique de formule générale IV

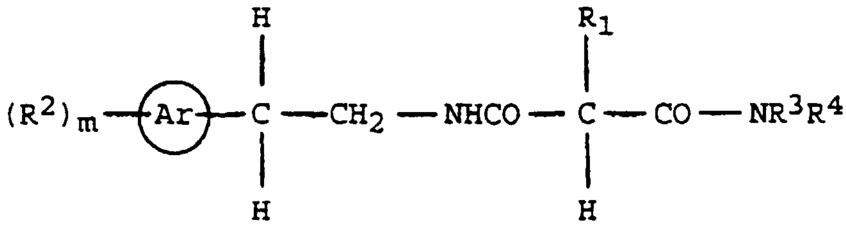

où $R^1$, $R^2$, $R^3$, $R^4$ et m sont définis comme dans l'une des revendications 1 à 15 et Ar représente phényle, indolyle ou 2- ou 3-thiényle, en présence d'un agent de condensation et, si on le souhaite, on le convertit en son sel.

**17.** Préparation pharmaceutique contenant un composé selon l'une des revendications 1 à 15.

**18.** Procédé de production de préparations pharmaceutiques selon la revendication 17 caractérisé en ce que l'on traite un composé selon l'une des revendications 1 à 15 avec des adjuvants et/ou supports galéniques courants pour obtenir des formes d'utilisation pharmaceutiques courantes.

**19.** Utilisation d'un composé de formule I, tel qu'il est défini dans l'une des revendications 1 à 15, pour la préparation d'un agent cardioprotecteur, d'un agent cérébroprotecteur ou d'un agent pour le traitement des processus inflammatoires chroniques, d'un agent à effet antiprolifératif ou d'un agent pour le traitement de la colite ulcéreuse ou de la maladie de Crohn.